(19) Europäisches Patentamt
European Patent Office
Office européen des brevets

(11) EP 1 785 415 B1

(12) **EUROPEAN PATENT SPECIFICATION**

(45) Date of publication and mention of the grant of the patent:
**02.05.2012 Bulletin 2012/18**

(21) Application number: **05781323.0**

(22) Date of filing: **31.08.2005**

(51) Int Cl.:
*C07D 211/16* (2006.01) *C07D 211/18* (2006.01)
*C07D 211/22* (2006.01) *C07D 211/28* (2006.01)
*C07D 211/34* (2006.01) *C07D 211/42* (2006.01)
*C07D 211/62* (2006.01) *C07D 401/06* (2006.01)
*C07D 401/12* (2006.01) *C07D 405/12* (2006.01)
*C07D 413/06* (2006.01) *C07D 471/18* (2006.01)
*C07D 487/08* (2006.01) *C07D 491/113* (2006.01)
*A61K 31/445* (2006.01)

(86) International application number:
**PCT/JP2005/015949**

(87) International publication number:
**WO 2006/025471 (09.03.2006 Gazette 2006/10)**

# (54) PIPERIDINE DERIVATIVE OR PHARMACEUTICALLY ACCEPTABLE SALT THEREOF

PIPERIDINDERIVAT ODER PHARMAZEUTISCH UNBEDENKLICHES SALZ DAVON

DÉRIVÉ DE PIPÉRIDINE OU SEL PHARMACEUTIQUEMENT ACCEPTABLE DE CELUI-CI

(84) Designated Contracting States:
**AT BE BG CH CY CZ DE DK EE ES FI FR GB GR HU IE IS IT LI LT LU LV MC NL PL PT RO SE SI SK TR**

(30) Priority: **01.09.2004 JP 2004254478**

(43) Date of publication of application:
**16.05.2007 Bulletin 2007/20**

(73) Proprietors:
- **Astellas Pharma Inc.**
  **Tokyo 103-8411 (JP)**
- **Kotobuki Pharmaceutical Co., Ltd.**
  **Hanishina-gun, Nagano 389-0697 (JP)**

(72) Inventors:
- **KIKUCHI, Kazumi**
  **c/o Astellas Pharma Inc.,**
  **Chuo-ku, Tokyo 1038411 (JP)**
- **FUJIYASU, Jiro**
  **c/o Astellas Pharma Inc.,**
  **Chuo-ku, Tokyo 1038411 (JP)**
- **WATANABE, Toshihiro**
  **c/o Astellas Pharma Inc.,**
  **Chuo-ku, Tokyo 1038411 (JP)**
- **NAGAKURA, Yukinori**
  **c/o Astellas Pharma Inc**
  **Chuo-ku, Tokyo 1038411 (JP)**
- **TOMIYAMA, Hiroshi**
  **Kotobuki Pharmaceutical Co., Ltd**
  **Hanishina-gun, Nagano 3890697 (JP)**
- **SONEGAWA, Motoharu**
  **Kotobuki Pharmaceutical Co.Ltd**
  **Hanishina-gun, Nagano 3890697 (JP)**
- **TOKUZAKI, Kazuo**
  **Kotobuki Pharmaceutical Co., Ltd.**
  **Hanishina-gun, Nagano 3890697 (JP)**
- **IWAI, Yoshinori**
  **Kotobuki Pharmaceutical Co., Ltd.**
  **Hanishina-gun, Nagano 3890697 (JP)**

(74) Representative: **HOFFMANN EITLE**
**Patent- und Rechtsanwälte**
**Arabellastraße 4**
**81925 München (DE)**

(56) References cited:
**EP-A- 1 254 904** **EP-A- 1 602 645**
**WO-A-2004/011430** **WO-A1-01/53288**
**WO-A1-2004/011430** **WO-A1-2004/078715**
**JP-A- 2 000 179** **JP-A- 2002 523 448**

- **LAMONTAGNE MP ET AL: 'Antimalarials. 8. Synthesis of amino ethers as candidate antimalarials.' J MED CHEM. vol. 19, no. 3, 1976, pages 360 - 365, XP002993309**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

Technical Field

The present invention relates to a novel piperidine derivative or a pharmaceutically acceptable salt thereof, as well as to a medicinal composition. More particularly, the present invention relates to a novel piperidine derivative or a pharmaceutically acceptable salt thereof, both having an excellent sodium channel inhibition action and an excellent analgesic action, as well as to a medicinal composition containing the above-mentioned piperidine derivative or pharmaceutically acceptable salt thereof and a pharmaceutically acceptable carrier, and more particularly, to a pharmaceutical composition having an analgesic action with reduced side effects, especially on neuropathic pain which acts as a sodium channel inhibitor.

Background Art

A voltage-dependent sodium channel is a protein responsible for the initiation and propagation of action potentials in neurons. The voltage-dependent sodium channel is composed of one larger $\alpha$ subunit with four domains, each consisting of six transmembrane segments, as a common structure and two smaller $\beta$ subunits. A major part of the channel function is played by $\alpha$ subunit. To date, more than 10 different $\alpha$ subunit subtypes have been known (Goldin AL, Annals of the New York Academy of Sciences 868:38-50, 1999). Each voltage-dependent sodium channel subtype shows distinct distributions in the central and peripheral nerve tissues. These subtypes regulate neural excitability and play an important role in regulating physiological functions in individual tissues. It is also suggested that they are deeply associated with various pathological conditions (Goldin AL, Annual Review of Physiology 63:871-894, 2001).
In recent years, it has become clear that voltage-dependent sodium channels are deeply involved in neural transmission of pain, and sodium channel inhibitors are expected to be excellent pain therapeutics, especially neuropathic pain therapeutics (Taylor CP, Current Pharmaceutical Design 2: 375-388, 1996).

Neuropathic pain means a pain that results from dysfunction in the central or peripheral neurons and refers to painful diabetic neuropathy, cancer pain, trigeminal neuralgia, phantom limb pain, postherpetic neuralgia, thalamic pain, etc. The clinical picture of neuropathic pain includes stabbing pain, burning pain, hyperalgesia, allodynia, etc. In medical scenes, non-steroidal anti-inflammatory drugs, narcotic analgesics such as morphine, etc. are used for the purpose of relieving pain. Recently, antiarrhythmic drugs and anticonvulsants, which are sodium channel inhibitors, have come to be used as well, for the purpose of relieving pain.

The non-steroidal anti-inflammatory drugs are not fully satisfactory in analgesic effect and further have a problem of side effects (e.g. gastrointestinal disorder and renal disorder). The narcotic analgesics (e.g. morphine) are highly effective mainly for nociceptive pain but have a problem of big side effects on digestive system, respiratory system and central nervous system. Further, these drugs have, in general, a small meritorious effect on neuropathic pain. Conventional sodium channel inhibitors, i.e. anti-arrhythmic drugs (e.g. lidocaine and, mexiletine) and anticonvulsant drugs (e.g. carbamazepine) have come to be used also for pain alleviation. However, these sodium channel inhibitors have central side effects (e.g. convulsion and drowsiness) and peripheral side effects (e.g. brachycardia) and, therefore, have had problems in that administration at a sufficiently high dose is difficult, making it difficult to obtain a sufficient analgesic effect.

As described above, no analgesic has been found yet which is effective for treatment of neurogenic pain and yet highly safe. Therefore, there is desired a novel sodium channel inhibitor which is highly effective particularly for neuropathic pain and has a low side effect. A WO 01/53288 pamphlet (hereinafter referred to as Patent Literature 1) describes a sodium channel inhibitor represented by the following general formula:

[formula 1]

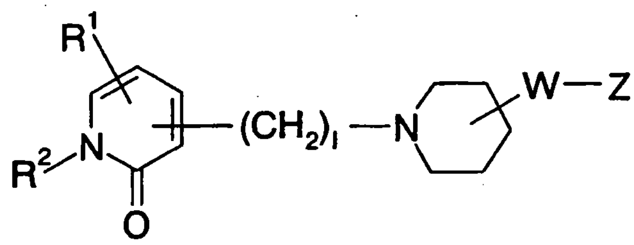

[in the above formula, the symbol (W) is a $C_{1-6}$ alkylene group which may be substituted, or the like; the symbol (Z) is a $C_{6-14}$ aromatic hydrocarbon ring group which may be substituted, or the like; the symbol (1) is 0 or an integer of 1 to 6; the symbols ($R^1$) and ($R^2$) are each hydrogen atom, or the like. For the details of these symbols, reference is made to the Patent Literature 1.] The compound disclosed in the Patent Literature 1 is a compound in which the piperidine ring is bonded, via a lower alkylene group or the like [the symbol (W)], to an aromatic hydrocarbon ring group or the like [the symbol (Z)] and the 1-position of the piperidine ring is bonded, via a lower alkylene, to an oxodihydropyridine ring. Meanwhile, the compound of the present invention is different from the compound disclosed in the Patent Literature 1, in the basic skeleton in that the 4-position of the piperidine ring is bonded, vi vinylene, to a mono-substituted ($-R^1$) or unsubstituted benzene ring and the 1-position of the piperidine ring has an acyl group [$-C(=O)-R^4$].

A WO 94/13291 pamphlet (hereinafter referred to as Patent Literature 2) discloses a nitrogen-containing heterocyclic ring derivative having styryl group (-CH=CH-benzene ring) represented by the following general formula:

[formula 2]

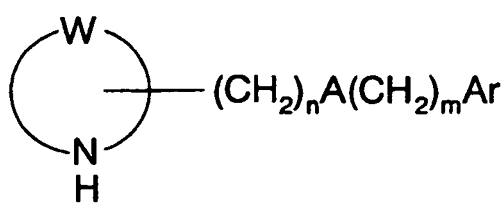

[in the above formula, the symbol (W) is $-(CH_2)_4-$, $-(CH_2)_5-$, $-(CH_2)_2O(CH_2)_2-$ or $-(CH_2)_2S(CH_2)_2-$; the symbol (A) is a bond, -CH=CH-, O, S, $NR^1$ or the like; the symbol ($R^1$) is hydrogen atom, $C_{1-3}$ alkyl or phenyl-$C_{1-3}$ alkyl; the symbol (Ar) is aryl or hetero-aryl; the symbol (n) is an integer of 0 to 6; and the symbol (m) is an integer of 0 to 3]. Incidentally, for the details of these symbols, reference is made to the Patent Literature 2.

A WO 97/19059 pamphlet (hereinafter referred to as Patent Literature 3) discloses a nitrogen-containing heterocyclic ring derivative represented by the following general formula:

[formula 3]

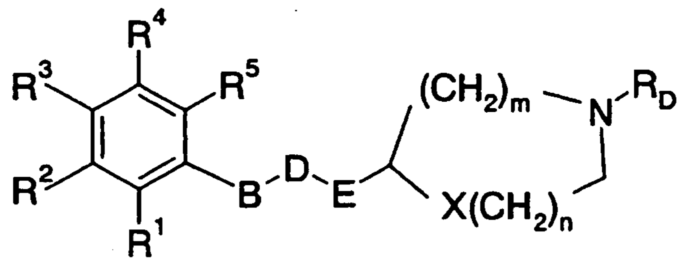

[in the above formula, the symbol (B) is not present or is lower alkylene, cycloalkylene or the like; the symbol (D) is

O-, -S-, -C(O)-, -C(O)-O-, -S(O)-, $-S(O)_2-$ or the like; the symbol (E) is lower alkylene or the like; the symbol (X) is not present or is -O-, -S- or the like; the symbols ($R^1$) to ($R^5$) are each hydrogen atom, halogen or the like; the symbol ($R_D$) is hydrogen atom, lower alkyl or the like; the symbol (n) is an integer of 0 to 3; and the symbol (m) is an integer of 0 to 2]. Incidentally, for the details of these symbols, reference is made to the Patent Literature 3.

In the Patent Literatures 2 and 3, however, there is neither disclosure nor suggestion on a compound such as the piperidine derivative of the present invention, in which the 4-position of the piperidine ring is bonded, vi vinylene, to a mono-substituted ($-R^1$) or unsubstituted benzene ring and the 1-position of the piperidine ring has an acyl group [-C (=O)-$R^4$]. Further, the applications of the compounds of the Patent Literatures 2 and 3 are a calcium channel antagonist (the Patent Literature 2) and a promoter for acetylcholine release (the Patent Literature 3) and, in these Patent Literatures, neither mention nor suggestion is made on sodium channel inhibition action or analgesic action.

Disclosure of the Invention

The present invention aims at providing a novel piperidine derivative or a pharmaceutically acceptable salt thereof, having an excellent sodium channel inhibition action and an excellent analgesic action, and a medicinal composition containing a novel piperidine derivative or a pharmaceutically acceptable salt thereof; particularly, a sodium channel inhibition compound showing a high analgesic effect to neurogenic pain and having a low side effect, and a medicinal composition containing the compound as an active ingredient.

The present inventors made a study on nitrogen-containing heterocyclic ring derivatives. As a result, it was found that a piperidine derivative in which the 4-position of the piperidine ring is bonded, via vinylene, to a mono-substituted ($-R^1$) or unsubstituted benzene ring and the 1-position of the piperidine ring has an acyl group [-C(=O)-$R^4$], or a pharmaceutically acceptable salt thereof shows a high inhibition action (activity) to sodium channel and further shows a good analgesic action to mice of streptozotocin-induced diabetic nerve disorder as a morbid state animal model. The finding has led to the completion of the present invention. According to the present invention, there are provided a novel compound and a medicinal composition containing the compound as an active ingredient, both described below.

[1] A piperidine derivative represented by Formula (I) or a pharmaceutically acceptable salt thereof:

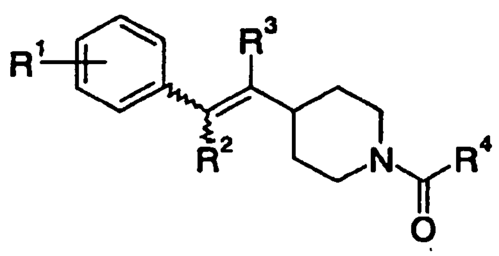

(I)

wherein $R^1$ is a hydrogen atom, halogen atom, $C_{1-6}$ alkyl which may be substituted, -O-$C_{1-6}$ alkyl which may be substituted, -O-aryl, aryl, cycloalkyl, -C(=O)-$C_{1-6}$ alkyl, -COOH, -C(=O)-O-$C_{1-6}$ alkyl, $-C(=O)-NH_2$, -C(=O)NH-$C_{1-6}$ alkyl, -C(=O) N-($C_{1-6}$ alkyl)$_2$, -OH, -O-C(=O)-$C_{1-6}$ alkyl, $NH_2$, -NH-$C_{1-6}$ alkyl, -N-($C_{1-6}$ alkyl)$_2$, -NH-C(=O)-$C_{1-6}$ alkyl, -CN or $NO_2$;$R^2$ and $R^3$ may be the same or different and are each a hydrogen atom, $C_{1-6}$ alkyl or halogen atom; and $R^4$ is one of mono-valent groups (a), (b) and (c):

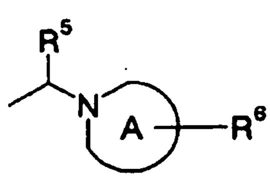

(a)

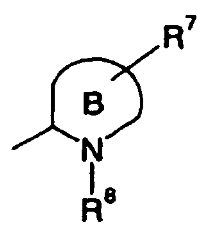

(b)

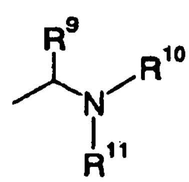

(c)

wherein A and B are each a nitrogen-containing heterocyclic ring; $R^5$ and $R^8$ to $R^{11}$ may be the same or different and are each a hydrogen atom, $C_{1-6}$ alkyl, -C(=O)-O-$C_{1-6}$ alkyl which may be substituted, $C_{1-6}$ alkylene-O-$C_{1-6}$ alkyl, cycloalkyl or a saturated or unsaturated 5- or 6-membered heterocyclic ring having 1 to 3 hetero-atoms selected from N, S and

O; $R^6$ is hydrogen atom, $C_{1-6}$ alkyl, -O-$C_{1-6}$ alkyl, -O-$C_{1-6}$ alkylene-O- which is bonded with one carbon atom of A ring to form a ring, -C(=O)-O-$C_{1-6}$ alkyl which may be substituted, -OH, -$C_{1-6}$ alkylene-OH, or -C(=O)-hetero-aryl; and $R^7$ is hydrogen atom, $C_{1-6}$ alkyl, -O-$C_{1-6}$ alkyl, -C(=O) -O-$C_{1-6}$ alkyl, -OH, -$C_{1-6}$ alkylene-OH or -C(=O)-hetero-aryl.

[2] A piperidine derivative or a pharmaceutically acceptable salt thereof according to [1], wherein $R^4$ is the mono-valent group (a) and the nitrogen-containing heterocyclic ring represented by A is a pyrrolidine, piperidine, morpholine, piperazine or oxazepam ring.

[3] A piperidine derivative or a pharmaceutically acceptable salt thereof according to [1], wherein $R^4$ is the mono-valent group (b) and the nitrogen-containing heterocyclic ring represented by B is a pyrrolidine or piperidine ring.

[4] A piperidine derivative or a pharmaceutically acceptable salt thereof according to [1], wherein $R^4$ is the mono-valent group (c) and $R^9$ to $R^{11}$ may be the same or different and are each hydrogen atom, $C_{1-6}$ alkyl, -C(=O)-O-$C_{1-6}$ alkyl which may be substituted, $C_{1-6}$ alkylene-O-$C_{1-6}$ alkyl, cycloalkyl or a saturated or unsaturated, 5- or 6-membered heterocyclic ring group having 1 to 3 hetero-atoms selected from N, S and O.

[5] A piperidine derivative or a pharmaceutically acceptable salt thereof according to [4], wherein at least one of $R^9$ to $R^{11}$ in the group (c) is cycloalkyl and the other(s) may each independently be a hydrogen atom, $C_{1-6}$ alkyl, -C (=O)-O-$C_{1-6}$ alkyl which may be substituted, lower $C_{1-6}$-O-$C_{1-6}$ alkyl, cycloalkyl or a saturated or unsaturated 5- or 6-membered heterocyclic ring group having 1 to 3 hetero-atoms selected from N, S and O.

[6] A piperidine derivative or a pharmaceutically acceptable salt thereof according to [1], wherein the piperidine derivative represented by the Formula (I) is:

4-(2-{4-[(E)-2-(2-methylphenyl)vinyl]piperidin-1-yl}-2-oxoethyl)morpholine;
4-(2-{4-[(E)-2-(4-isopropylphenyl)vinyl]piperidin-1-yl}-2-oxoethyl)morpholine;
4-(2-{4-[(E)-2-(4-chlorophenyl)vinyl]piperidin-1-yl}-2-oxoethyl)morpholine;
N-(2-{4-[(E)-2-(4-chlorophenyl)vinyl]piperidin-1-yl}-2-oxoethyl)cyclohexaneamine; or
N-(2-{4-[(E)-2-(4-chlorophenyl)vinyl]piperidin-1-yl}-2-oxoethyl)-N-methylcyclohexaneamine.

[7] A composition containing a piperidine derivative or a pharmaceutically acceptable salt thereof according to any of [1] to [6] and a pharmaceutically acceptable carrier.

[8] A composition according to [7] for use in the treatment of pain.

The piperidine derivative or pharmaceutically acceptable salt thereof, of the present invention has been confirmed to have an excellent sodium channel inhibition action and an excellent analgesic action and show a high analgesic effect particularly to neurogenic pain. Therefore, the present compound is useful as a sodium channel inhibitor of low side effect.

## Best Mode for Carrying Out the Invention

The piperidine derivative or pharmaceutically acceptable salt thereof, of the present invention is described specifically. Description is made first on the definition of each symbol used in the general formula (I) and the mono-valent groups (a), (b) and (c), as well as on specific examples of each symbol.

The terms $C_{1-6}$ alkyl or $C_{1-6}$ alkylene indicate, unless otherwise specified, a straight chain or branched chain hydrocarbon chain having 1 to 6 carbon atoms. As $C_{1-6}$ alkyl there can be mentioned, for example, $C_{1-6}$ alkyls such as methyl, ethyl, propyl, isopropyl, butyl, isobutyl, sec-butyl, tert-butyl, pentyl, isopentyl, neopentyl, tert-pentyl, hexyl, isohexyl and the like; and there are preferred methyl, ethyl, propyl, butyl and tert-butyl.

As $C_{1-6}$ alkylene, there can be mentioned, for example, methylene, ethylene, propylene and isopropylene; and methylene and ethylene are preferred. The term "cycloalkyl" indicates a mono- to tri-cyclic, aliphatic saturated hydrocarbon ring group having 3 to 14 carbon atoms, and there can be mentioned for example, cyclopropyl, cyclobutyl, cyclopentyl, cyclohexyl, cycloheptyl, cyclooctyl, bicycloheptyl, bicyclooctyl, bicyclononyl, bicyclodecanyl, tricyclononyl, tricyclodecanyl, tricycloundecanyl and tricyclododecanyl, and there are preferred cyclopropyl, cyclobutyl, cyclopentyl, cyclohexyl, cycloheptyl and cyclooctyl.

The term "aryl" indicates a mono- to tri-cyclic, aromatic hydrocarbon group having 6 to 14 carbon atoms; and there can be mentioned, for example, phenyl, naphthyl, anthryl and phenanthryl, and phenyl and naphthyl are preferred.

The term "hetero-aryl" indicates hetero-aryl having 1 to 3 hetero-atoms selected from N, S and O, and is preferably pyridyl and pyrimidyl. The term "nitrogen-containing heterocyclic ring" indicates a mono- or di-cyclic, nitrogen-containing hetero-aryl ring having 5 to 10 atoms including 1 to 3 nitrogen atoms, and may further include 1 to 3 oxygen or sulfur atoms besides the nitrogen atom(s). There can be mentioned, for example, pyrrole, imidazole, pyrazole, triazole, tetrazole, pyridine, pyridazine, pyrimidine, pyrazine, triazine, indoline, isoindoline, benzimidazoline, benzopyrazoline, pyrrolopyridine, imidazopyridine, quinoline, isoquinoline and quinoxaline. The term "nitrogen-containing heterocyclic ring" also indicates a mono- or di-cyclic, nitrogen-containing hetero-cycloalkyl having 3 to 10 atoms including 1 to 3 nitrogen atoms, and there can be mentioned, for example, aziridine, azetidine, pyrrolidine, piperidine, piperazine, hexahydroazepine, quinuclidine, azabicyclooctane (e.g. azabicyclo[3.2.1]octane), diazabicyclooctane, azabicyclononane and azabicyclodecane. There may be included, besides the nitrogen atom(s), 1 to 3 oxygen or sulfur atoms and there can be mentioned morpholine, oxazepam, oxazole, isooxazole, thiazole, isothiazole, furazane, etc. The "nitrogen-containing heterocyclic ring" is preferably pyrrolidine, piperidine, morpholine or oxazepam ring. As the "nitrogen-containing heterocyclic ring group", there can be mentioned mono-valent groups of above-mentioned "nitrogen-containing heterocyclic rings".

As "saturated or unsaturated, 5- or 6-membered heterocyclic ring group having 1 to 3 hetero-atoms selected from N, S and O", there can be mentioned tetrahydropyranyl, furanyl, thiophenyl, pyrrolyl and morpholyl. The group includes part of above-mentioned "nitrogen-containing heterocyclic ring groups" and is preferably tetrahydropyranyl or morpholinyl.

As "halogen atom", there can be mentioned fluorine, chlorine, bromine and iodine; and fluorine and chlorine are preferred.

In the term expressing the substituent "$C_{1-6}$ alkyl which may be substituted", the expression "which may be substituted" indicates "which may be substituted with same or different, 1 to 3 substituents". The "$C_{1-6}$ alkyl which may be substituted", is preferably a substituted methyl group represented by the following formula.

[Formula 6]

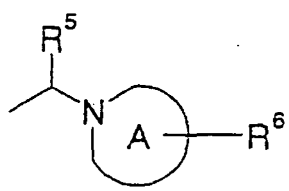

, or

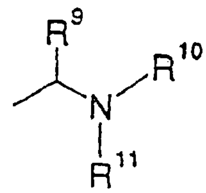

In the compound (I) of the present invention, there are optical isomers (e.g. optical active compounds and diastereomers) or geometrical isomers, depending upon the kinds of substituents. Therefore, the present compound (I) includes mixtures of these optical isomers or geometrical isomers, and isolated compounds.

Also, the present compound (I) can form an acid addition salt or a salt with a base. As such a salt, there can be mentioned, for example, addition salts with an inorganic acid such as hydrochloric acid, hydrobromic acid, hydriodic acid, sulfuric acid, nitric acid, phosphoric acid or the like; addition salts with an organic acid such as formic acid, acetic acid, propionic acid, oxalic acid, malonic acid, succinic acid, fumaric acid, maleic acid, lactic acid, malic acid, citric acid, tartaric acid, carbonic acid, picric acid, methanesulfonic acid, ethanesulfonic acid, glutamic acid or the like; salts with an inorganic base such as sodium, potassium, magnesium, calcium, aluminum or the like; and salts with an organic salt such as methylamine, ethylamine, monoethanolamine, diethanolamine, triethanolamine, cyclohexylamine, lysine, ornithine or the like. Further, the present compound (I) or the pharmaceutically acceptable salt thereof can form, in some cases, a hydrate, a solvate (e.g. a solvate with ethanol) or a polymorphism.

Furthermore, the compound (I) of the present invention includes all compounds (that is, prodrugs) which can be metabolized and converted, in a living body, into a present compound (I) or a pharmaceutically acceptable salt thereof. As the group capable of forming a prodrug of the present compound (I), there can be mentioned, for example, groups described in Prog. Med. 5:2157-2161 (1985) and groups described in "Development of Drugs" (published by Hirokawa Shoten in 1990) Vol. 7 (Molecular Design) 163-198. These groups are specifically those which can be converted into the primary amine, secondary amine, OH, HOC(=O)- or the like of the present invention, by hydrolysis or solvolysis or under physiological conditions. As prodrugs of OH, there can be mentioned, for example, lower alkyl-COO- which may be substituted, aryl-C(=O)O- which may be substituted, ROC(=O)-substituted or unsubstituted lower alkylene-C(=O)O-(R is H- or lower alkyl. The same applies hereinafter), ROC(=O)-substituted or unsubstituted lower alkenylene-C(=O)O-, ROC(=O)-lower alkylene-0-lower alkylene-C(=O)O-, ROC(=O)-C(=O)O-, $ROS(=O)_2$-substituted or unsubstituted lower alkenylene-C(=O)O-, phthalidyl-O-, and 5-methyl-1,3-dioxolen-2-on-4-yl-methyloxy.

Hereinafter, description is made on the representative production processes of the present compound (I), the synthesis of raw materials, and the recipes.

[Production processes]

The present compound (I) can be produced by various synthesis processes utilizing the characteristics based on the basic skeleton or the kinds of substituents. Here, two production processes (the first production process and the

second production process) are described.

(First production process)

The first production process is a process for producing the present compound (I) according to the reaction path shown below.

[Formula 8]

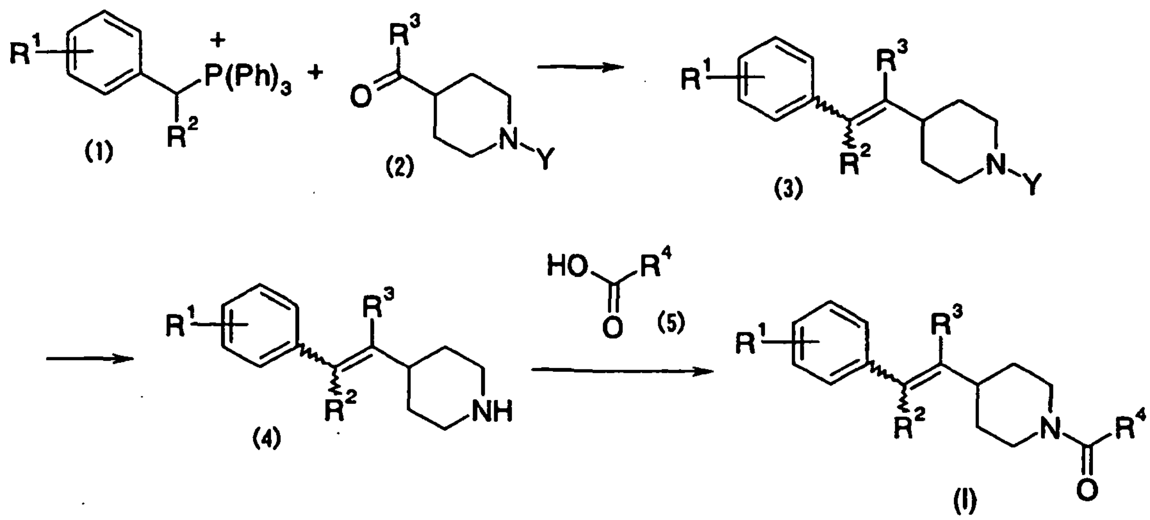

(1) (2) (3) (4) (5) (I)

In the above reaction path, the symbols ($R^1$) to ($R^4$) indicate the above-mentioned mono-valent groups. The symbol (P) indicates phosphorus atom; the symbol (Ph) indicates phenyl group; and the symbol (Y) indicates protecting group for amino. The same applies hereinafter.

According to the first production process, the compound (I) of the present invention can be easily obtained by conducting, by an ordinary method, a Wittig reaction between a phosphonium salt (1) and an aldehyde or ketone (2) [Org. React., 14, 270-490 (1965); WO 01/53288] to obtain a compound (3), removing the amino-protecting group of the compound (3) to obtain a compound (4), and conducting amidation between the compound (4) and a carboxylic acid (5). As the solvent for the Wittig reaction, there can be used an organic solvent not participating in the reaction, such as tetrahydrofuran, dioxane, dimethyl sulfoxide, toluene or the like. As the base, there can be used sodium hydride, potassium tert-butoxide, sodium ethoxide, lithium diisopropylamide, or the like. The reaction can be conducted at a temperature from -70°C to the refluxing temperature. As the amino-protecting group, there can be mentioned tert-butoxycarbonyl group, benzyloxycarbonyl group, etc. The deprotection (removal of protecting group) can be conducted by ordinary deprotection (Protective Group in Organic Synthesis, second ed., JOHN WILEY & SONS, INC.). The successive amidation can be conducted by an ordinary method.

(Second production process)

The second production process is a process for producing the present compound (I) according to the reaction path shown below.

[Formula 9]

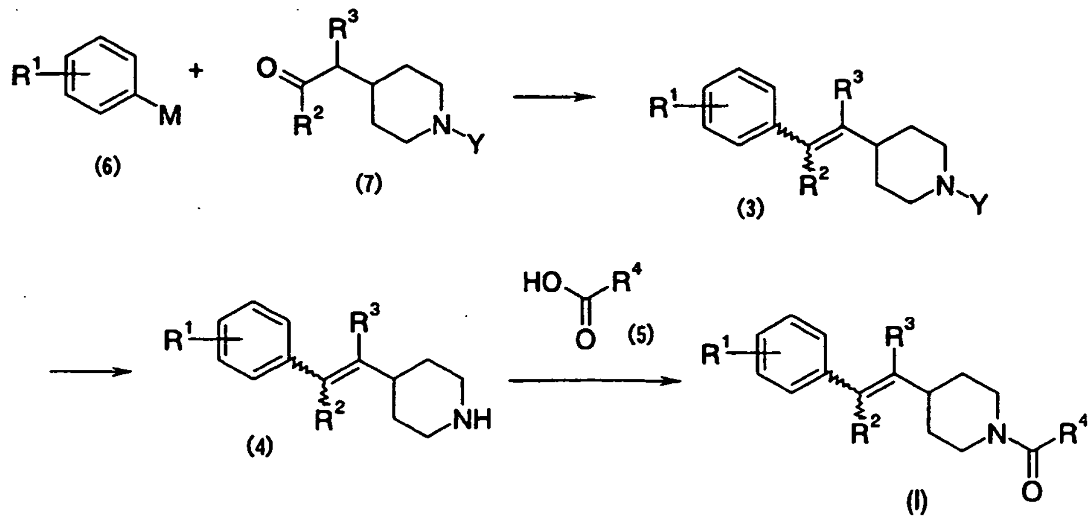

(6) (7) (3) (4) (5) (I)

In the above reaction path, the symbols ($R^1$) to ($R^4$) indicate the above-mentioned mono-valent groups; the symbol (M) indicates Li, MgCl or the like; and the symbol (Y) indicates an amino-protecting group. The same applies hereinafter.

According to the second production process, the present compound (I) can be obtained by an ordinary reaction between an aryl metal (e.g. aryl lithium or aryl Grignard) (6) and a carbonyl compound (7) [Org. Synth. III, 200 (1955); Org. React., 6, 339-366 (1964); Org. React., 8, 258-304 (1967)]. As the reaction solvent, there can be used an organic solvent not participating in the reaction, such as diethyl ether, tetrahydrofuran, dioxane, dimethyl sulfoxide, toluene or the like. The reaction can be conducted at a temperature from -70˚C to the refluxing temperature. The successive removal of amino-protecting group and amidation can be conducted in the same manner as in the first production process.

The present compound (I) can also be obtained by the reactions other than the above-mentioned reactions, for example, Peterson reaction [Org. React., 38, 1-223 (1990)] and triple bond formation followed by partial reduction [J. Am. Chem. Soc., 77, 3378 (1955); J. Am. Chem. Soc., 99, 2805 (1977); Synthesis, 1973, 457; and Tetrahedron 30, 3817 (1974)].

[Synthesis of raw materials] The raw materials for the present compound (I) can be produced easily according to the synthesis processes described in the above-mentioned literatures [Org. React., 14, 270-490 (1965); WO 01/53288; Org. React., 16, 1-438 (1968); Org. Synth., III, 200 (1955); Org. React., 6, 339-366 (1964); Org. React., 8, 258-304 (1967)] and Org. Chem. 43, 4099 (1978).

The thus produced compound (I) of the present invention is isolated as a free form or as a pharmaceutically acceptable salt thereof. The salt of the present compound (I) can be produced by subjecting the present compound (I) (which is a free base) to an ordinary salt formation reaction.

Also, the present compound (I) or the pharmaceutically acceptable salt thereof is isolated and purified as its hydrate, solvate, or polymorphism. The isolation and purification is conducted by applying ordinary chemical operations such as extraction, concentration, distillation, crystallization, filtration, recrystallization, various chromatographies and the like.

Various isomers can be separated by using appropriately selected raw materials, or by utilizing the differences in physical or chemical properties between isomers. For example, optical isomers can be purified into stereochemically pure isomers by using appropriately selected raw materials or by racemic resolution of racemic compounds (for example, the racemic compounds are converted into diastereomer salts with an ordinary optically active acid, followed by optical resolution).

[Recipes]

To the present compound (I) can be applied various recipes used generally. Representative recipes applicable to the present compound (I) are described below.

The medicinal composition of the present invention containing at least one kind of present compound (I) or pharmaceutically acceptable salt thereof, of the present invention can contain a pharmaceutically acceptable carrier.

By using a carrier, a filler and other additives, all employed ordinarily in pharmaceutical preparations, the present medicinal composition is prepared in the form of tablets, powder, parvules, granules, capsules, pills, solution, injection, suppositories, ointment, paps or the like, and is administered orally (includes sublingual administration) or parenterally.

The clinical dose of the present compound (I) or its pharmaceutically acceptable salt to humans is appropriately determined in each individual case in consideration of the symptom, weight, age and sex of an individual patient, the route of administration, etc.; however, the administration is ordinarily conducted orally in a total amount of 1 mg to 1,000 mg, preferably 10 mg to 200 mg in one to several times per adult per day, or intravenously in an amount of 1 mg to 500 mg in one to several times per adult per day, or intravenously administered in a sustained release manner for a period of 1 hour to 24 hours per day. Since the dose differs depending upon various conditions as described above, an amount smaller than the above dose may be sufficient.

As the solid composition of the present invention for oral administration, there are used tablets, a powder, granules, etc. In such a solid composition, one or more active substances are mixed with at least one kind of inactive diluent such as lactose, mannitol, glucose, hydroxypropylcellulose, microcrystallized cellulose, starch, polyvinylpyrrolidone, magnesium metasilicate aluminate or the like. The composition may contain, according to the conventional method, additives other than the inactive diluents, for example, a lubricant such as magnesium stearate, a disintegrator such as starch, or cellulose calcium glycolate, a stabilizer like lactose, and a solubilizer such as glutamic acid or aspartic acid. The tablets or pills may be sugar-coated or coated with a gastric soluble or enteric film, using sucrose, gelatin, hydroxypropylcellulose and hydroxypropylmethylcellulose phthalate, and the like.

The liquid composition for oral administration contains an emulsifier, a solvent, a suspending agent, a syrup, an elixir, etc. , which are all pharmaceutically acceptable, and further contains an inactive diluent ordinarily used, such as purified water, ethanol or the like. This composition may contain, besides the inactive diluents, an auxiliary agent such as solubilizer, wetting agent or suspending agent, a sweetening agent, a flavoring agent, an aromatic agent, a preservative, etc.

The injection for parenteral administration contains a solubilizer, a suspending agent or an emulsifier, which are all sterile and aqueous or non-aqueous. The aqueous solubilizer or suspending agent includes, for example, a distilled water for injection and a physiological saline solution. The non-aqueous solubilizer or suspending agent includes, for example, propylene glycol, polyethylene glycol, a vegetable oil such as olive oil, an alcohol such as ethanol and Polysolvate 80 (trade name). Such a composition may further contain additives such as an isotonic agent, a preservative, a wetting agent, an emulsifier, a dispersing agent, a stabilizer such as lactose, a solubilization- and dissolution-auxiliary agent and the like. The composition is sterilized by, for example, filtration through a bacteria retention filter, or by blending with sterilizers or irradiation. Alternatively, it may also be prepared into an aseptic solid composition and the resulting aseptic composition is provided for use after dissolution in aseptic water or in an aseptic solvent for injections, prior to use. The present compound (I) may be used in combination with a therapeutic for the diseases described above or with other drugs useful for pain by a mechanism other than the sodium channel blockage. A drug useful for pain, which is usable in combination, includes narcotic analgesics, antipyretic analgesics, non-steroidal antiinflammatory drugs, etc.

## Examples

The present invention is described in more detail below by way of Examples [Production Examples of present compound (I)]. However, the present invention is in no way restricted to these Examples. First, production examples of the raw materials used in the following Examples are described as Reference Examples.

### (Reference Example 1)

To 15.0 ml of an N,N-dimethylformamide suspension containing 2.38 g of (2-chlorobenzyl)(triphenyl)phosphonium chloride was added 0.63 g of potassium tert-butoxide. The mixture was stirred at room temperature for 10 minutes. To the resulting orange suspension was added 1.00 g of tert-butyl 4-formylpiperidine-1-carboxylate, and the resultant was stirred for 15 minutes for reaction. The resulting reaction mixture was poured into a saturated aqueous ammonium chloride solution, and extracted with ethyl acetate. The organic layer was washed with brine and then dried over anhydrous sodium sulfate. After filtration, the filtrate was concentrated under reduced pressure. The residue was purified on silica gel column chromatography (n-hexane-ethyl acetate) to obtain 1.45 g of tert-butyl 4-[2-(2-chlorophenyl)vinyl]piperidine-1-carboxylate as a colorless oil.

### (Reference Examples 2 to 32)

Compounds shown in Tables 1 to 3 were obtained in the same manner as in Reference Example 1.

(Reference Example 33)

To 1.45 g of tert-butyl 4-[2-(2-chlorophenyl)vinyl]piperidine-1-carboxylate was added 2.10 g of p-toluenesulfonic acid. The mixture was stirred at 150˚C for 5 hours for reaction. To the resulting reaction mixture was added 10.0 ml of water. The mixture was adjusted to ca. pH 11 with 10% aqueous sodium hydroxide solution, and extracted with chloroform. The organic layer was washed with water and brine and then dried over anhydrous sodium sulfate. After filtration, the filtrated was concentrated under reduced pressure to obtain 0.97 g of 4-[(E)-2-(2-chlorophenyl)vinyl]piperidine as a yellow oil.

(Reference Examples 34 to 40)

Compounds shown in Table 3 were obtained in the same manner as in Reference Example 33.

(Reference Example 41)

5.0 ml of a chloroform solution containing 0.55 g of 4-[(E)-2-(3-chlorophenyl)vinyl]piperidine and 0.5 ml of triethylamine was added to 5.0 ml of a chloroform solution containing 0.2 ml of chloroacetyl chloride, and the resultant was stirred at room temperature for 20 minutes for reaction. The reaction mixture was poured into a 10% hydrochloric acid. The organic layer was separated, and washed with a saturated aqueous sodium hydrogencarbonate solution and brine and then dried over anhydrous sodium sulfate. After filtration, the filtrate was concentrated under reduced pressure. The residue was purified on silica gel column chromatography (n-hexane-ethyl acetate) to obtain 0.42 g of 1-(chloroacetyl)-4-[(E)-2-(3-chlorophenyl)vinyl]piperidine as a yellow oil.

(Reference Examples 42 to 45)

Compounds shown in Tables 3 and 4 were obtained in the same manner as in Reference Example 41.

(Example 1)

To 10.0 ml of an N,N-dimethylformamide solution containing 0.45 g of 4-[(E)-2-(2-chlorophenyl)vinyl]piperidine were added 0.37 g of morpholin-4-yl-acetic acid mono-hydrochloride, 0.39 g of 1-ethyl-3-(3-dimethylaminopropyl)carbodiimide mono-hydrochloride, 0.27 g of 1-hydroxybenztriazole mono-hydrate and 0.28 ml of triethylamine. The mixture was stirred at room temperature for 2 days for reaction. The resulting reaction mixture was poured into a 10% aqueous potassium carbonate solution, and extracted with ethyl acetate. The organic layer was washed with water and brine and then dried over anhydrous sodium sulfate. After filtration, the filtrate was concentrated under reduced pressure. The residue was purified on silica gel column chromatography (ethyl acetate) to obtain 0.56 g of 4-(2-{4-[(E)-2-(2-chlorophenyl) vinyl]piperidin-1-yl}-2-oxoethyl)morpholine as pale yellow crystals. These crystals were made into a solution of 3.0 ml of ethanol and 1.0 ml of tetrahydrofuran. Thereto was added 2.0 ml of an ethanol solution containing 0.14 g of oxalic acid. The resulting crystals were collected by filtration and recrystallized from ethanol to obtain 0.52 g of 4-(2-{4-[(E)-2-(2-chlorophenyl)vinyl]piperidin-1-yl}-2-oxoethyl)morpholine oxalate as colorless powdery crystals.

(Examples 2 to 54)

Compounds shown in Tables 5 to 13 were obtained in the same manner as in Example 1.

(Example 55)

To 10.0 ml of an N,N-dimethylformamide solution containing 0.80 g of 1-(chloroacetyl)-4-[(E)-2-(4-chlorophenyl) vinyl]piperidine were added 0.41 g of piperidin-3-ol and 0.74 g of potassium carbonate, and the resultant was stirred at room temperature for 6 hours for reaction. The resulting reaction mixture was poured into a 10% aqueous potassium carbonate solution, and extracted with ethyl acetate. The organic layer was washed with brine and then dried over anhydrous sodium sulfate. After filtration, the filtrate was concentrated under reduced pressure. The residue was purified on silica gel column chromatography (ethyl acetate-ethanol) to obtain 0.92 g of 1-(2-{4-[(E)-2-(4-chlorophenyl)vinyl] piperidin-1-yl}-2-oxoethyl)piperidin-3-ol as a colorless oil. This oil was dissolved in 5.0 ml of ethanol. Thereto was added 5.0 ml of an ethanol solution of containing 0.23 g of oxalic acid. The resulting crystals were collected by filtration and recrystallized from ethanol-ethyl acetate to obtain 0.96 g of 1-(2-{4-[(E)-2-(4-chlorophenyl)vinyl]piperidin-1-yl}-2-oxoethyl)piperidin-3-ol oxalate as colorless powdery crystals.

(Examples 56 to 65)

Compounds shown in Tables 13 to 15 were obtained in the same manner as in Example 55.

(Comparative Example 66)

To 20.0 ml of an N,N-dimethylformamide solution containing 2.00 g of 1-(chloroacetyl)-4-[(E)-2-(4-chlorophenyl)vinyl]piperidine was added 1.86 g of potassium phthalimide, and the resultant was stirred at 50°C for 3 hours for reaction. The resulting reaction mixture was poured into a 10% aqueous potassium carbonate solution, extracted with chloroform. The organic layer was washed with water and brine and then dried over anhydrous sodium sulfate. After filtration, the filtrate was concentrated under reduced pressure. The residue was purified on silica gel column chromatography (chloroform-acetone) to obtain 2.46 g of 2-(2-{4-[(E)-2-(4-chlorophenyl)vinyl]piperidin-1-yl}-2-oxoethyl)-1H-isoindole-1,3(2H)-dione as colorless powdery crystals.

(Example 67)

To 50.0 ml of a methanol suspension containing 2.46 g of 2-(2-{4-[(E)-2-(4-chlorophenyl)vinyl]piperidin-1-yl}-2-oxoethyl)-1H-isoindole-1,3(2H)-dione was added 0.65 ml of hydrazine mono-hydrate, and the resultant was refluxed for 1.5 hours. The resulting reaction mixture was cooled to room temperature and concentrated under reduced pressure. To the residue was added 100.0 ml of a 5% aqueous sodium hydroxide solution, and extracted with chloroform. The organic layer was washed with water and brine and then dried over anhydrous sodium sulfate. After filtration, the filtrate was concentrated under reduced pressure to obtain 1.80 g of 2-{4-[(E)-2-(4-chlorophenyl)vinyl]piperidin-1-yl}-2-oxoethaneamine as a colorless oil.

(Example 68)

To 5.0 ml of ethanol solution containing 0.71 g of tert-butyl 4-(2-{4-[(E)-2-(3-chlorophenyl)vinyl]piperidin-1-yl}-2-oxoethyl)piperazine-1-carboxylate was added 10.0 ml of 35% hydrochloric acid-ethanol solution. The mixture was stirred at room temperature for 2 hours for reaction. The resulting reaction mixture was concentrated under reduced pressure. The residue was azeotropically distilled with 5.0 ml of ethanol three times. The resulting residue was recrystallized from ethanol to obtain 0.46 g of 1-(2-{4-[(E)-2-(3-chlorophenyl)vinyl]piperidin-1-yl}-2-oxoethyl)piperazine di-hydrochloride as colorless crystals.

(Examples 69 to 74)

Compounds shown in Tables 15 to 16 were obtained in the same manner as in Example 68.

(Example 75)

To 10.0 ml of an N,N-dimethylformamide suspension containing 0.70 g of 1-(2-{4-[(E)-2-(4-chlorophenyl)vinyl]piperidin-1-yl}-2-oxoethyl)piperazine di-hydrochloride were added 0.46 ml of triethylamine and 0.56 g of nicotinic chloride mono-hydrochloride. The mixture was stirred at room temperature for 2 hours for reaction. The resulting reaction mixture was poured into a 10% aqueous potassium carbonate solution, and extracted with ethyl acetate. The organic layer was washed with water and brine and then dried over sodium sulfate. After filtration, the filtrate was concentrated under reduced pressure. The residue was purified on silica gel column chromatography (n-hexane-ethyl acetate) to obtain 0.73 g of 1-(2-{4-[(E)-2-(4-chlorophenyl)vinyl]piperidin-1-yl}-2-oxoethyl)-4-(pyridin-3-ylcarbonyl)piperazine as a pale yellow oil. To 3.0 ml of ethanol solution containing it was added 2.0 ml of an ethanol solution containing 0.15 g of oxalic acid. The resulting crystals were collected by filtration and recrystallized from ethanol to obtain 0.66 g of 1-(2-{4-[(E)-2-(4-chlorophenyl)vinyl]piperidin-1-yl}-2-oxoethyl)-4-(pyridin-3-ylcarbonyl)piperazine oxalate as opaque white powdery crystals.

(Example 76)

To 20.0 ml of methylene chloride solution containing 1.80 g of 2-{4-[(E)-2-(4-chlorophenyl)vinyl]piperidin-1-yl)-2-oxoethaneamine were added 0.67 ml of cyclohexanone and 0.35 ml of acetic acid to obtain a solution of about pH 5. The solution was stirred at room temperature for 15 minutes. Thereto was added 1.37 g of sodium triacetoxyborohydride, and the resultant was stirred for 10 minutes for reaction. The resulting reaction mixture was poured into a 10% aqueous potassium carbonate solution, and extracted with chloroform. The organic layer was washed with brine and then dried

over anhydrous sodium sulfate. After filtration, the filtrate was concentrated under reduced pressure. The residue was purified on silica gel column chromatography (chloroform-ethanol) to obtain 1.73 g of N-2-{4-[(E)-2-(4-chlorophenyl) vinyl]piperidin-1-yl}-2-oxoethyl)cyclohexaneamine as light brown crystals. 10.0 ml of ethanol solution was prepared by dissolving 0.88 g of the compound. To the solution was added 5.0 ml of ethanol solution containing 0.22 g of oxalic acid. The resulting crystals were collected by filtration and recrystallized from ethanol-ethyl acetate to obtain 1.0 g of N-2-{4-[(E)-2-(4-chlorophenyl)vinyl]piperidin-1-yl}-2-oxoethyl)cyclohexaneamine oxalate as colorless powdery crystals.

(Example 77)

Compounds shown in Table 17 were obtained in the same manner as in Example 76.

(Example 78)

To 10.0 ml of acetonitrile solution containing 0.81 g of N-2-{4-[(E)-2-(4-chlorophenyl)vinyl]piperidin-1-yl}-2-oxoethyl)cyclohexaneamine was added 1.0 ml of 37% formaldehyde solution, at room temperature, and the resultant was stirred for 5 minutes. Thereto was added 0.15 ml of acetic acid, and stirred for 10 minutes for reaction. 0.85 g of sodium triacetoxyborohydride was added, the resultant was stirred for 10 minutes. The resulting reaction mixture was poured into a 10% aqueous potassium carbonate solution, and extracted with ethyl acetate. The organic layer was washed with brine and then dried over anhydrous sodium sulfate. After filtration, the filtrate was concentrated under reduced pressure. The residue was purified on alumina column chromatography (n-hexane-ethyl acetate) to obtain 0.69 g of N-2-{4-[(E)-2-(4-chlorophenyl)vinyl]piperidin-1-yl}-2-oxoethyl)-N-methylcyclohexaneamine as a colorless oil. After preparing 5.0 ml of ethanol solution of it, 5.0 ml of ethanol solution containing 0.16 g of oxalic acid was added thereto. The resulting crystals were collected by filtration and recrystallized from ethanol-ethyl acetate to obtain 0.72 g of N-2-{4-[(E)-2-(4-chlorophenyl)vinyl]piperidin-1-yl}-2-oxoethyl)-N-methylcyclohexaneamine oxalate as colorless powdery crystals.

(Example 79)

Compounds shown in Table 17 were obtained in the same manner as in Example 78.

(Example 80)

6 ml of a 20% aqueous sodium hydroxide solution was added to 30 ml of methanol solution containing 2.85 g of methyl 3-{(E)-2-[1-(morpholin-4-ylacetyl)piperidin-4-yl]vinyl}benzoate. The mixture was refluxed under heating for 1 hour, distilled under reduced pressure to remove the solvent. To the residue was added 10% hydrochloric acid for neutralization. The resulting mixture was extracted with chloroform and the extract was dried over anhydrous sodium sulfate. The dried extract was distilled under reduced pressure to remove the solvent. After the resulting crystals were suspended in ethyl acetate, the resulting suspension was filtered to collect 1.91 g of 3-{(E)-2-[1-(morpholin-4-ylacetyl) piperidin-4-yl]vinyl}benzoic acid.

(Examples 81 to 82)

Compounds shown in Table 17 were obtained in the same manner as in Example 80.

(Example 83)

To 8 ml of acetonitrile suspension containing 589 mg of 4-{(E)-2-[1-(morpholin-4-ylacetyl)piperidin-4-yl]vinyl} benzoic acid were added 943 mg of 1-ethyl-3-(3-dimethylaminopropyl)carbodiimide mono-hydrochloride, 556 mg of 1-hydroxybenztriazole and 1.5 ml of a 50% aqueous dimethylamine solution. The mixture was refluxed under heating for 1.5 hours. After cooling, the reaction mixture was concentrated under reduced pressure. To the residue was added a saturated aqueous sodium hydrogencarbonate solution, and the resultant was extracted with chloroform. The extract was washed with brine and then dried over anhydrous sodium sulfate. The dried extract was distilled under reduced pressure to remove the solvent. The residue was purified on silica gel column chromatography (chloroform-methanol) to obtain 315 mg of N,N-dimethyl-4-{(E)-2-[1-(morpholin-4-ylacetyl)piperidin-4-yl]vinyl}benzamide as a pale yellow oil. After preparing 8 ml of ethyl acetate solution of it, 4 ml of ethanol solution containing 74 mg of oxalic acid were added thereto. The resulting crystals were collected by filtration and recrystallized from ethanol-ethyl acetate to obtain 290 mg of N,N-dimethyl-4-{(E)-2-[1-(morpholin-4-ylacetyl)piperidin-4-yl]vinyl}benzamide oxalate.

(Examples 84 to 88)

Compounds shown in Table 18 were obtained in the same manner as in Example 83.

(Example 89)

6.5 ml of a 1.2 M methyl lithium-diethyl ether solution was added, at -78˚C, to 20 ml of tetrahydrofuran solution containing 824 mg of methyl 4-{(E)-2-[1-(morpholin-4-ylacetyl)piperidin-4-yl]vinyl}benzoate. The mixture was stirred at the same temperature for 3 hours. Thereto was added 20 ml of a saturated aqueous ammonium chloride solution, and the resultant was heated to room temperature. The reaction mixture was extracted with chloroform. The extract was washed with brine and then dried over anhydrous sodium sulfate. The dried extract was distilled under reduced pressure to remove the solvent. The residue was purified on silica gel column chromatography (ethyl acetate-methanol) to obtain 283 mg of 2-4-{(E)-2-[1-(morpholin-4-ylacetyl)piperidin-4-yl]vinyl}phenyl)propan-2-ol as a pale yellow oil. After 5 ml of ethyl acetate solution of it was prepared, 1 ml of ethanol solution of 68 mg of oxalic acid was added thereto. The resulting crystals were collected by filtration and recrystallized from ethanol to obtain 219 mg of 2-4-{(E)-2-[1-(morpholin-4-ylacetyl) piperidin-4-yl]vinyl}phenyl)propan-2-ol oxalate.

(Example 90)

To 20 ml of methanol solution containing 667 mg of 4-(2-{4-[(E)-2-(4-nitrophenyl)vinyl]piperidin-1-yl}-2-oxoethyl) morpholine were added 5 ml of water, 0.5 g of ammonium chloride and 0.5 g of a zinc powder. The mixture was refluxed under heating for 30 minutes. After cooling, the reaction mixture was filtered. The filtrate was concentrated under reduced pressure. To the residue was added water, and was extracted with ethyl acetate. The extract was washed with brine and then dried over anhydrous sodium sulfate. The dried extract was distilled under reduced pressure. The residue was purified on silica gel column chromatography (ethyl acetate-methanol) to obtain 600 mg of 4-{(E)-2-[1-morpholin-4-ylacetyl]piperidin-4-yl}vinyl}aniline as a pale yellow oil. 250 mg of the compound was made into a solution 2 ml of ethyl acetate and 1 ml of ethanol by dissolving it. To the resulting solution was added 1 ml of ethanol solution containing 68 mg of oxalic acid. The resulting crystals were collected by filtration and recrystallized from ethanol to obtain 231 mg of 4-{(E)-2-[1-morpholin-4-ylacetyl]piperidin-4-yl}vinyl}aniline oxalate.

(Example 91)

0.5 ml of acetic anhydride was added to 5 ml of pyridine solution containing 500 mg of 9-{(E)-2-[1-morpholin-4-ylacetyl)piperidin-4-yl]vinyl}aniline. The resulting mixture was stirred at room temperature for 6 hours for reaction. The reaction mixture was concentrated under reduced pressure. To the residue was added a 5% aqueous sodium hydroxide solution, and was extracted with ethyl acetate. The extract was washed with water and brine, and then dried over anhydrous sodium sulfate. The dried extract was distilled under reduced pressure to remove the solvent. To the residue was added ethyl acetate, and the resultant was stirred. The resulting crystals were collected by filtration to obtain 520 mg of N-4-{(E)-2-[1-(2-morpholin-4-ylacetyl)piperidin-4-yl]vinyl}phenyl)acetamide as pale yellow powdery crystals. After a solution of 3 ml of ethyl acetate and 3 ml of ethanol containing 450 mg of the compound, 1 ml of ethanol solution containing 110 mg of oxalic acid was added thereto. The resulting crystals were collected by filtration and recrystallized from ethanol to obtain 470 mg of N-4-{(E)-2-[1-(2-morpholin-4-ylacetyl)piperidin-4-yl]vinyl}phenyl)acetamide oxalate.

The chemical structural formulas and physical and chemical properties of the compounds obtained in the above Reference Examples, Comparative Example and Examples are shown in Tables 1 to 19. In addition to the compounds described in Examples, the compounds given in Tables 20 can be obtained by the processes described above, the processes described in Reference Examples and Examples, processes known to ordinary skill in the art and their modifications, without requiring any special experiments..

The symbols in the Tables indicate the followings.

Rf: Reference Example No., EX: Example No., C.Ex: Comparative Example No. Me: methyl group, MS: mass spectrum (unless otherwise specified, FAB or ESI) m/z, NMR: nuclear magnetic resonance spectrum (unless otherwise specified, 400 MHz, $^{1}$H-NMR, DMSO-$d_6$, TMS internal standard) $\delta$ (ppm)

Table 1

| Rf. | STRUCTURE | DATA | Rf. | STRUCTURE | DATA |
|---|---|---|---|---|---|
| 1 | 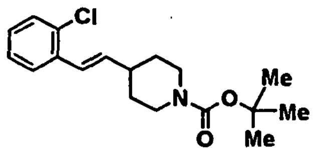 | MS: 321(EI) | 2 | 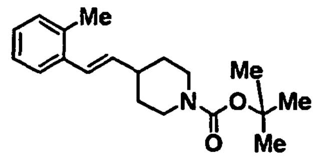 | MS: 301(EI) |
| 3 | 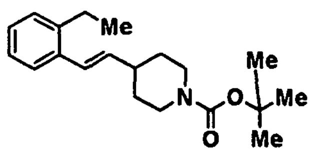 | MS: 315(EI) | 4 | 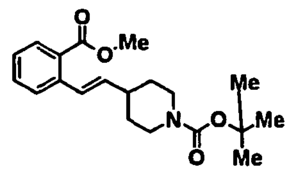 | MS: 345(EI) |
| 5 | 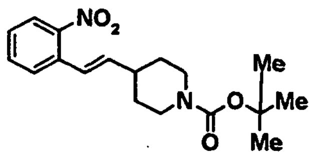 | MS: 332(EI) | 6 | 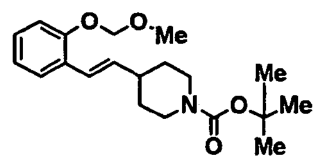 | MS: 347(EI) |
| 7 | 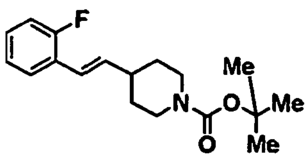 | MS: 305(EI) | 8 | 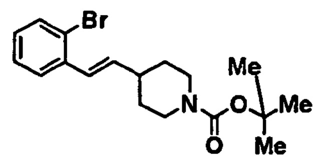 | MS: 365(EI) |
| 9 | 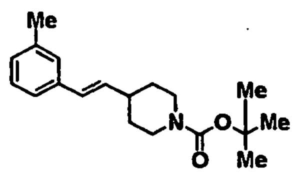 | MS: 301(EI) | 10 | 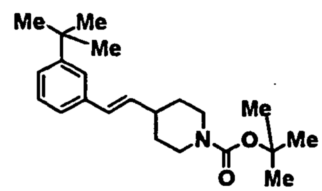 | MS: 343(EI) |
| 11 | 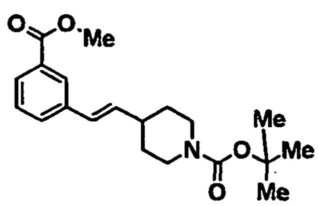 | MS: 345(EI) | 12 | 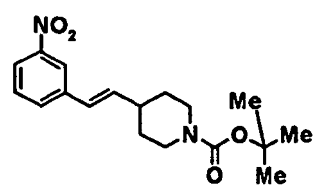 | MS: 332(EI) |
| 13 | 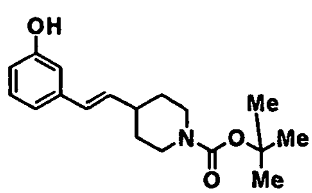 | MS: 303(EI) | 14 | 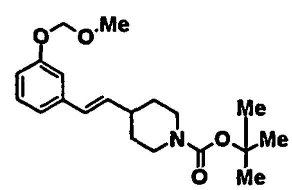 | MS: 347(EI) |

Table 2

| Rf. | STRUCTURE | DATA | Rf. | STRUCTURE | DATA |
|---|---|---|---|---|---|
| 15 | 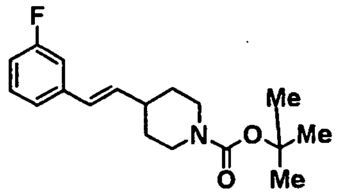 | MS: 305 (EI) | 16 | 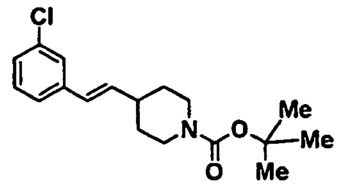 | MS: 321 (EI) |
| 17 | 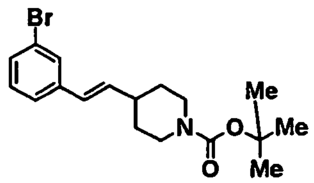 | MS: 365 (EI) | 18 | 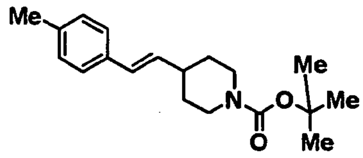 | MS: 301 (EI) |
| 19 | 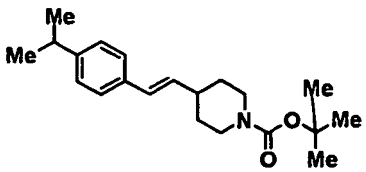 | MS: 329 (EI) | 20 | 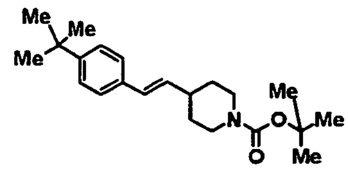 | MS: 343 (EI) |
| 21 | 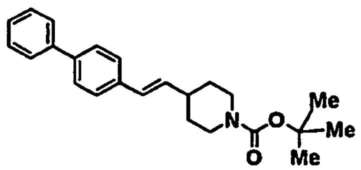 | MS: 363 (EI) | 22 | 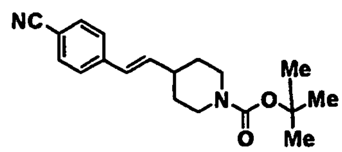 | MS: 312 (EI) |
| 23 | 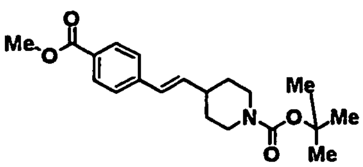 | MS: 345 (EI) | 24 | 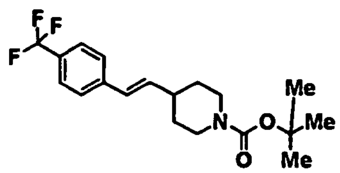 | MS: 355 (EI) |
| 25 | 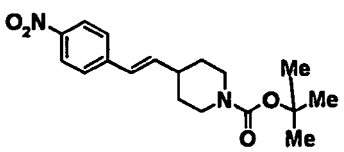 | MS: 332 (EI) | 26 | 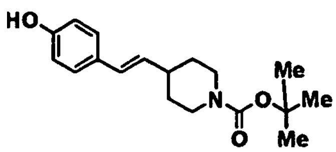 | MS: 303 (EI) |
| 27 | 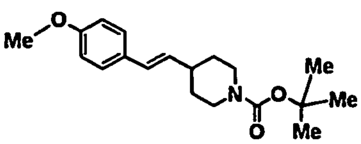 | MS: 317 (EI) | 28 | 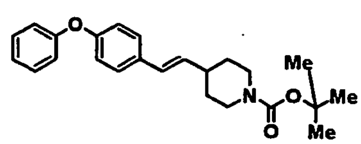 | MS: 379 (EI) |

Table 3

| Rf. | STRUCTURE | DATA | Rf. | STRUCTURE | DATA |
|---|---|---|---|---|---|
| 29 | 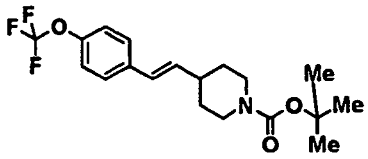 | MS: 371 (EI) | 30 | 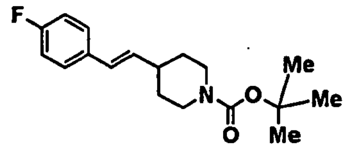 | MS: 305 (EI) |
| 31 | 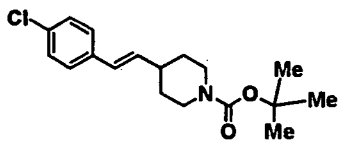 | MS: 321 (EI) | 32 | 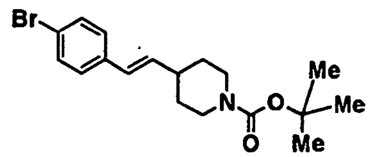 | MS: 365 (EI) |
| 33 | 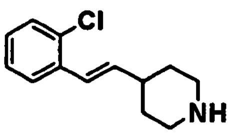 | MS: 222 | 34 | 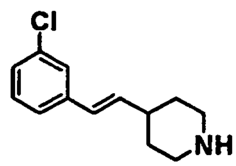 | MS:222 |
| 35 | 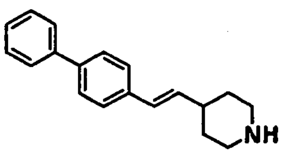 | MS:264 | 36 | 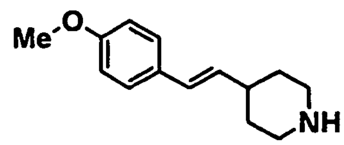 | MS:218 |
| 37 | 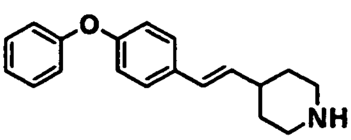 | MS: 280 | 38 | 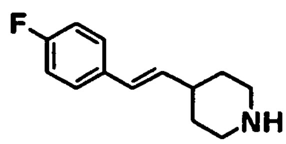 | MS:206 |
| 39 | 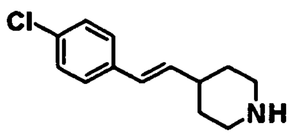 | MS:222 | 40 | 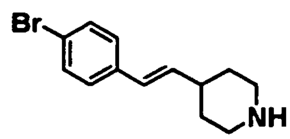 | MS: 265 (EI) |
| 41 | 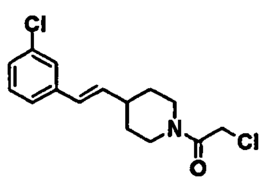 | MS: 297 (EI) | 42 | 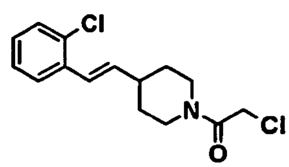 | MS: 297 (EI) |

Table 4

| Rf. | STRUCTURE | DATA | Rf. | STRUCTURE | DATA |
|---|---|---|---|---|---|
| 43 | 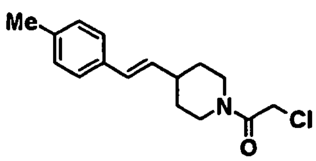 | MS: 278 | 44 | 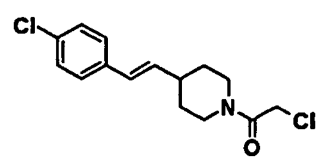 | MS: 297(EI) |

(continued)

| Rf. | STRUCTURE | DATA | Rf. | STRUCTURE | DATA |
|---|---|---|---|---|---|
| 45 | 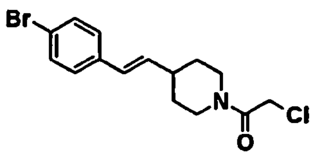 | MS: 343(EI) | | | |

Table 5

| Ex. | STRUCTURE | DATA |
|---|---|---|
| 1 | 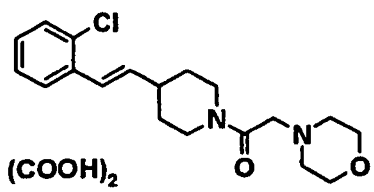 | NMR: 1.21 (1H, m), 1.41 (1H, m), 1.81 (2H, m), 2.50 (1H, m), 2.65-2.80 (5H, m), 3.45-3.80 (7H, m), 3.90 (1H, m), 4.37 (1H, m), 6.34 (1H, dd), 6.69 (1H, d), 7.20-7.35 (2H, m), 7.40 (1H, d), 7.66 (1H, d) MS: 349 |
| 2 | 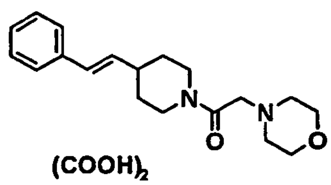 | MS: 315 |
| 3 | 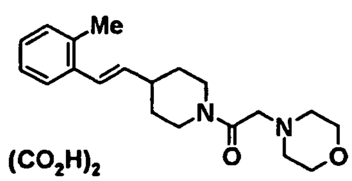 | NMR: 1.24 (1H, m), 1.40 (1H m), 1.81 (2H, m), 2.28 (3H, s), 2.47 (1H, m), 2.71 (1H, t), 2.80 (4H, m), 3.10 (1H, t), 3.51-3.80 (6H, m), 3.89 (1H, d), 4.37 (1H, d), 6.11 (1H, dd), 6.59 (1H, d), 7.12 (3H, m), 7.42 (1H, m) MS: 329 |
| 4 | 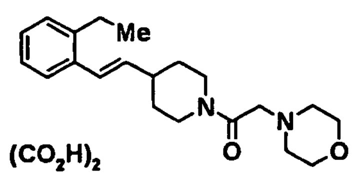 | MS: 343 |
| 5 | 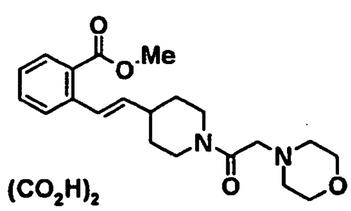 | MS: 373 |
| 6 | 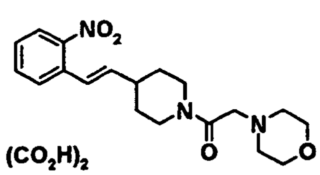 | MS: 360 |

Table 6

| Ex. | STRUCTURE | DATA |
|---|---|---|
| 7 | 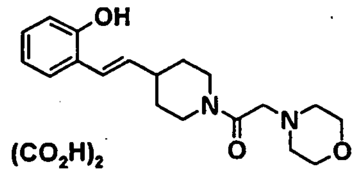 | MS: 331 |
| 8 | 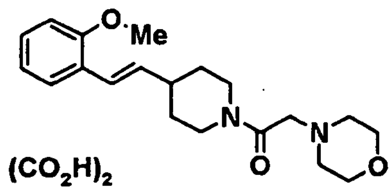 | MS: 345 |
| 9 | 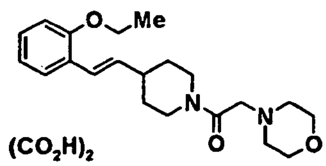 | MS: 359 |
| 10 | 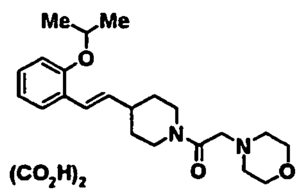 | MS: 373 |
| 11 | 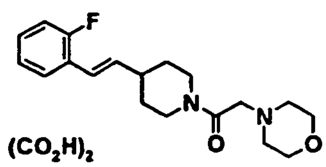 | MS: 333 |
| 12 | 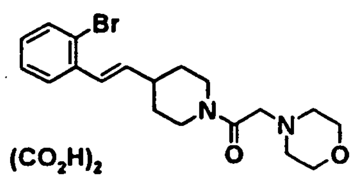 | MS: 395 |
| 13 | 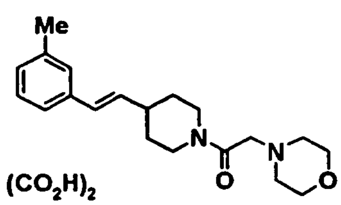 | MS: 329 |

Table 7

| Ex. | STRUCTURE | DATA |
|---|---|---|
| 14 | 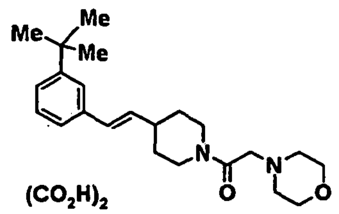 | MS: 371 |
| 15 | 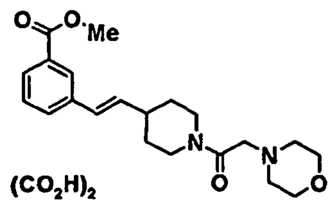 | MS: 373 |
| 16 | 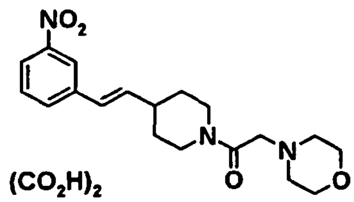 | MS: 360 |
| 17 | 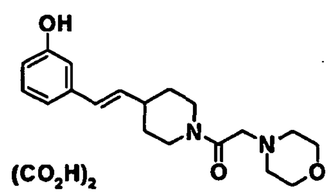 | MS: 331 |
| 18 | 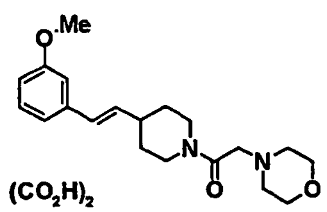 | MS: 345 |
| 19 | 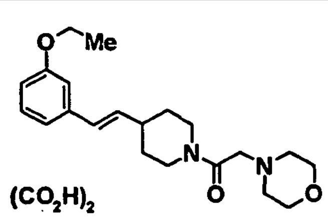 | MS: 359 |

Table 8

| Ex. | STRUCTURE | DATA |
|---|---|---|
| 20 | 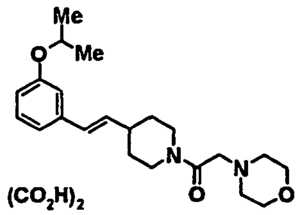 | MS: 373 |
| 21 | 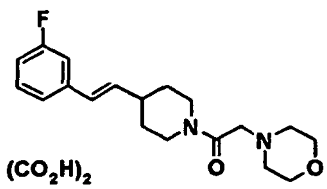 | MS: 333 |
| 22 | 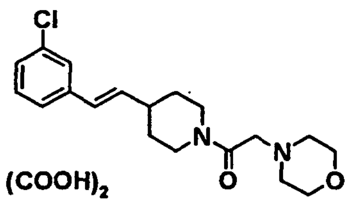 | NMR: 1.24 (1H, m), 1.37 (1H, m), 1.78 (2H, m), 2.42 (1H, m), 2.60-2.80 (5H, m), 3.08 (1H, m), 3.57 (1H, m), 3.60-3.75 (5H, m), 3.90 (1H, m), 4.35 (1H, m), 6.38 (2H, m), 7.23 (3H, m), 7.48 (1H, s) MS: 349 |
| 23 | 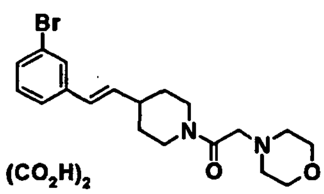 | MS: 395 |
| 24 | 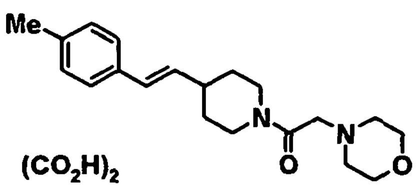 | NMR: 1.21 (1H, m), 1.37 (1H m), 1.87 (2H, m), 2.27 (3H, s), 2.41 (1H, m), 2.70 (1H, t), 2.85 (4H, m), 3.09 (1H, t), 3.68-3.87 (7H, m), 4.34 (1H, d), 6.18 (1H, dd), 6.34 (1H, d), 7.11 (2H, d), 7.28 (2H, d) MS: 329 |
| 25 | 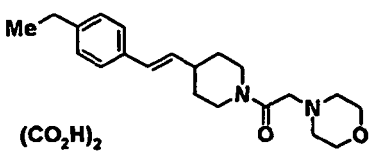 | MS: 343 |

Table 9

| Ex. | STRUCTURE | DATA |
|---|---|---|
| 26 | 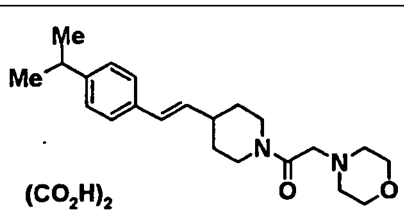 | NMR: 1.18 (6H, t), 1.17-1.39 (2H, m), 1.78 (2H, m), 2.40 (1H, m), 2.67-2.88 (6H, m), 3.08 (1H, t), 3.60-3.70 (6H, m), 3.87 (1H, d), 4.34 (1H, d), 6.18 (1H, dd), 6.35 (1H, d), 7.16 (2H, d), 7.29 (2H, d) MS: 357 |

(continued)

| Ex. | STRUCTURE | DATA |
|---|---|---|
| 27 | 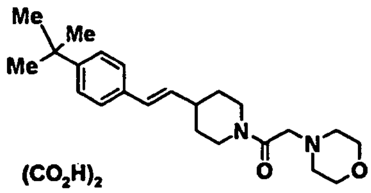 $(CO_2H)_2$ | NMR: 1.26 (9H, s), 1.26-1.39 (2H, m), 1.78 (2H, m), 2.42 (1H, m), 2.70 (1H, t), 2.79 (4H, m), 3.08 (1H, t), 3.64-3.89 (7H, m), 4.34 (1H, d), 6.18 (1H, dd), 6.35 (1H, d), 7.31 (4H, s) MS: 371 |
| 28 | 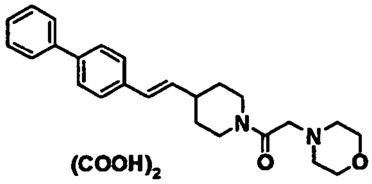 $(COOH)_2$ | MS: 391 |
| 29 | 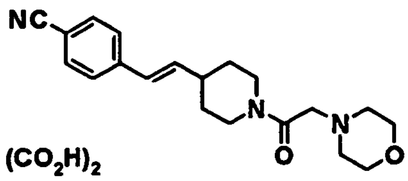 $(CO_2H)_2$ | MS: 340 |
| 30 | 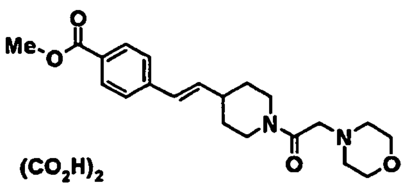 $(CO_2H)_2$ | MS: 373 |
| 31 | 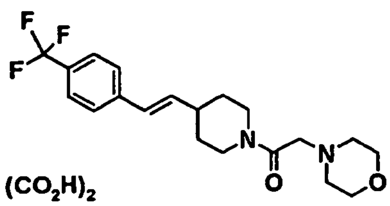 $(CO_2H)_2$ | MS: 383 |

Table 10

| Ex. | STRUCTURE | DATA |
|---|---|---|
| 32 | 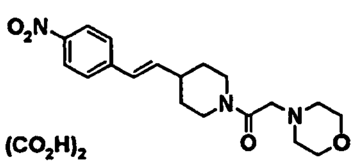 $(CO_2H)_2$ | MS: 360 |
| 33 | 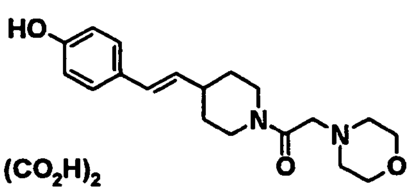 $(CO_2H)_2$ | MS: 331 |

(continued)

| Ex. | STRUCTURE | DATA |
|---|---|---|
| 34 | 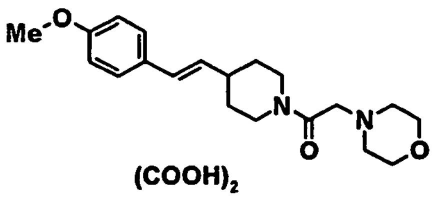 | MS: 345 |
| 35 | 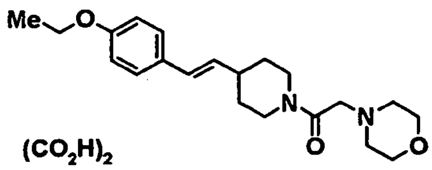 | MS: 359 |
| 36 | 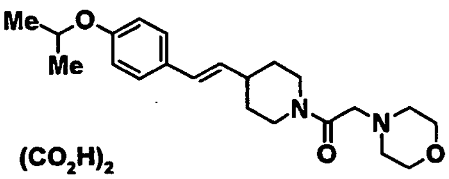 | MS: 373 |
| 37 | 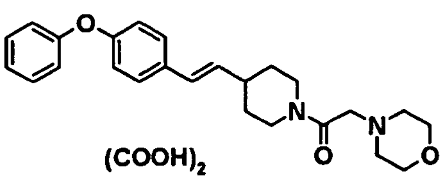 | NMR: 1.20-1.26 (1H, m), 1.34-1.42 (1H, m), 1.78 (2H, m), 2.42 (1H, m), 2.70 (1H, m), 2.75 (4H, m), 3.08 (1H, m), 3.57 (1H, m), 3.68-3.73 (5H, m), 3.89 (1H, m), 3.36 (1H, m), 6.19 (1H, dd), 6.40 (1H, d), 6.95 (2H, d) 6.99 (2H, dd), 7.13 (1H, t), 7.36-7.43 (4H, m) MS: 409 |

Table 11

| Ex. | STRUCTURE | DATA |
|---|---|---|
| 38 | 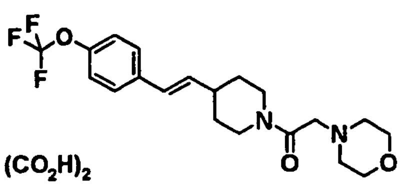 | MS: 399 |
| 39 | 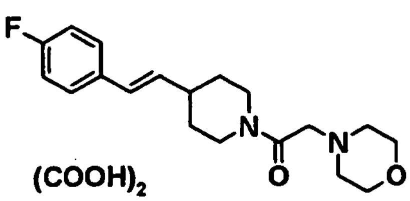 | MS: 333 |
| 40 | 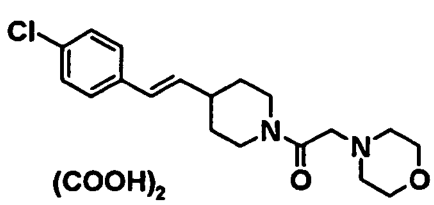 | NMR: 1.16-1.26 (1H, m), 1.33-1.42 (1H, m), 1.78 (2H, m), 2.42 (1H, m), 2.70 (1H, m), 2.79 (4H, m), 8.05 (1H, m), 3.58-3.69 (6H, m), 3.89 (1H, m), 4.36 (1H, m), 6.30 (1H, dd), 6.41 (1H, d), 7.36 (2H, d), 7.43 (2H, d) MS: 349 |

(continued)

| Ex. | STRUCTURE | DATA |
|---|---|---|
| 41 | 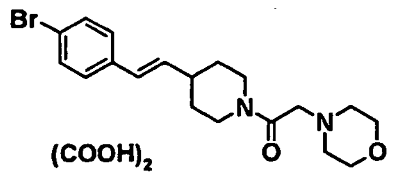 | MS: 395 |
| 42 | 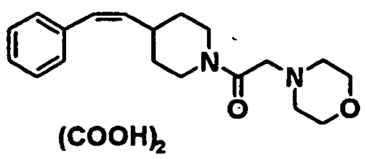 | MS: 315 |
| 43 | 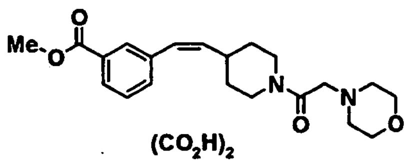 | MS: 373 |
| 44 | 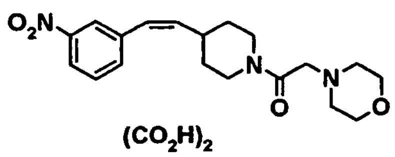 | MS: 360 |

Table 12

| Ex. | STRUCTURE | DATA |
|---|---|---|
| 45 | 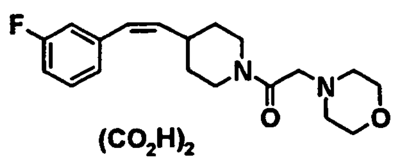 | MS: 333 |
| 46 | 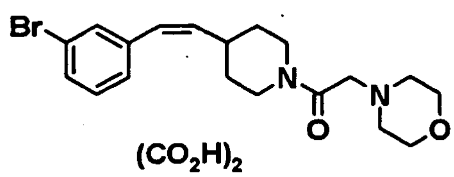 | MS: 395 |
| 47 | 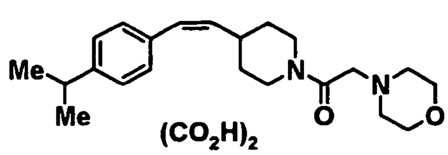 | MS: 357 |
| 48 | 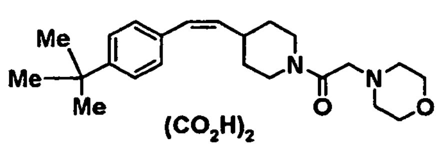 | MS: 371 |

(continued)

| Ex. | STRUCTURE | DATA |
|---|---|---|
| 49 | 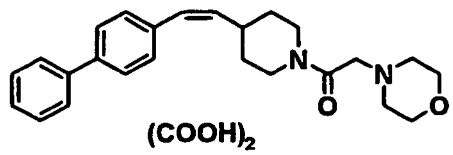 $(COOH)_2$ | MS: 391 |
| 50 | 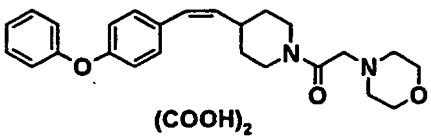 $(COOH)_2$ | MS: 409 |
| 51 | 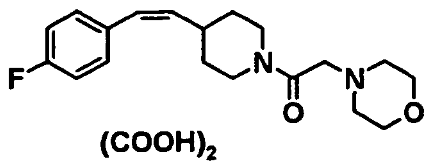 $(COOH)_2$ | MS: 333 |

Table 13

| Ex. | STRUCTURE | DATA |
|---|---|---|
| 52 | 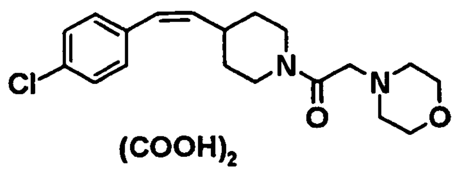 $(COOH)_2$ | MS: 349 |
| 53 | 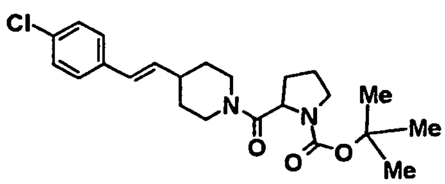 | MS: 418(EI) |
| 54 | 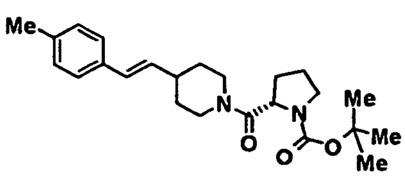 | MS: 399 |
| 55 | 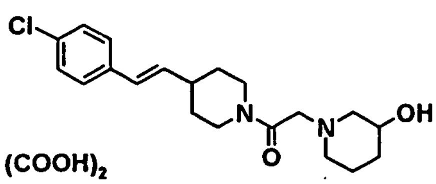 $(COOH)_2$ | NMR: 1.20-1.45 (3H, m), 1.64 (1H, m), 1.80 (4H, m), 2.45 (1H, m), 2.74 (2H, m), 3.09 (3H, m), 3.70-4.10 (4H, m), 4.36 (1H, m), 6.30 (1H, dd), 6.41 (1H, d), 7.36 (2H, d), 7.43 (2H, d) MS: 363 |
| 56 | 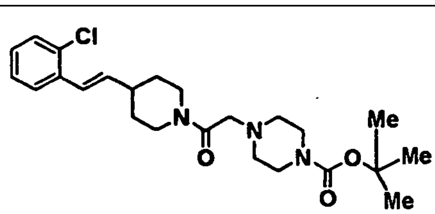 | MS: 447(EI) |

(continued)

| Ex. | STRUCTURE | DATA |
|---|---|---|
| 57 | 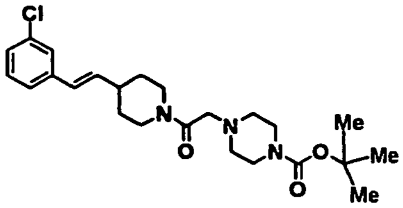 | |

Table 14

| Ex. | STRUCTURE | DATA |
|---|---|---|
| 58 | 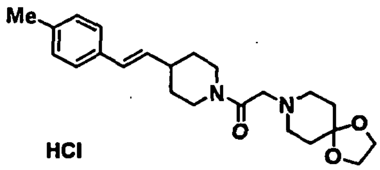 | MS: 385 |
| 59 | 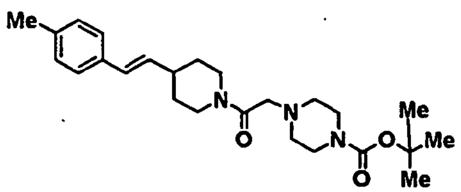 | MS: 428 |
| 60 | 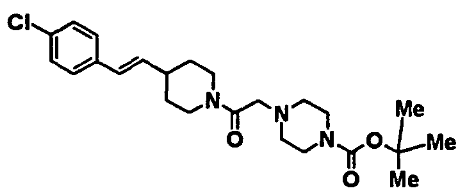 | MS: 447(EI) |
| 61 | 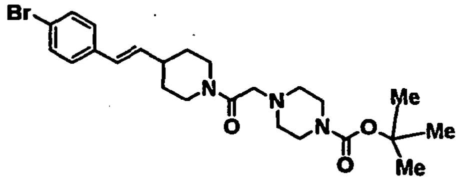 | MS: 493(EI) |
| 62 | 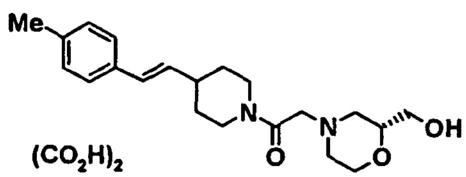 | MS: 359 |
| 63 | 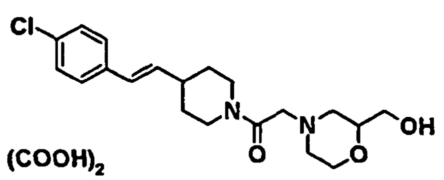 | MS: 379 |

Table 15

| Ex. | STRUCTURE | DATA |
|---|---|---|
| 64 | 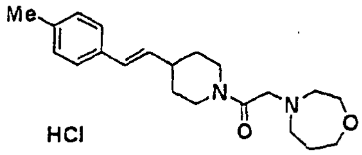 HCl | MS: 343 |
| 65 | 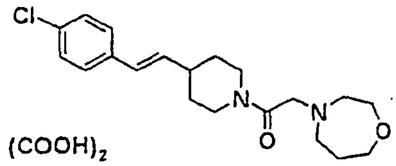 $(COOH)_2$ | MS: 363 |
| [C.Ex] 66 | 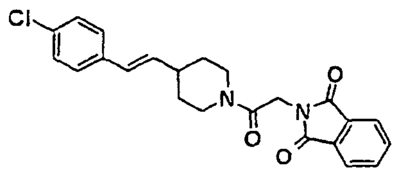 | MS: 408(EI) |
| 67 | 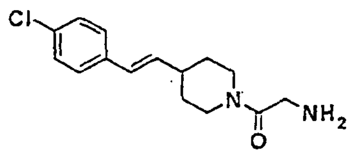 | MS: 278(EI) |
| 68 | 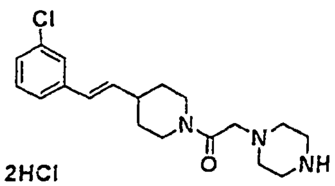 2HCl | NMR: 1.26 (1H, m), 1.41 (1H, m), 1.80 (2H, m), 2.46 (1H, m), 2.78 (1H, m), 3.11 (1H, m), 3.15-4.00 (10H, m), 4.35 (2H, m), 6.40 (2H, d), 7.27 (1H, d), 7.35 (2H, m), 7.49 (1H, s), 9.60· 10.00 (1H, m) MS: 348 |
| 69 | 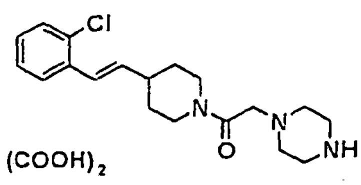 $(COOH)_2$ | MS: 348 |
| 70 | 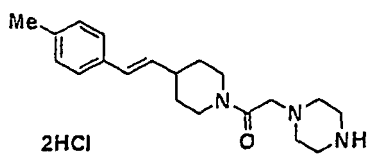 2HCl | MS: 328 |

Table 16

| Ex. | STRUCTURE | DATA |
|---|---|---|
| 71 | 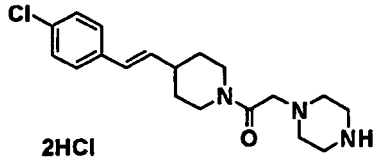 2HCl | MS: 348 |
| 72 | 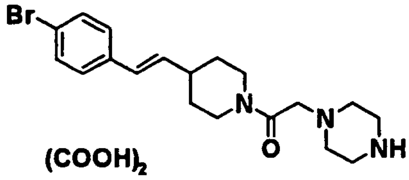 $(COOH)_2$ | MS: 394 |
| 73 | 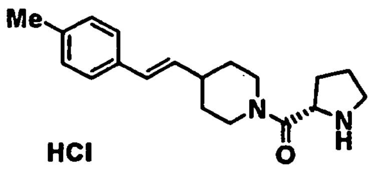 HCl | MS: 299 |
| 74 | 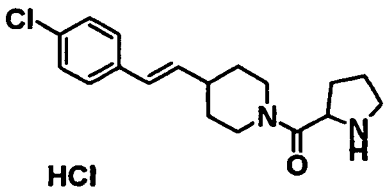 HCl | NMR: 1.20-1.50 (2H, m), 1.70-2.00 (5H, m), 2.45 (1H, m), 2.80 (1H, m), 3.10-3.35 (3H, m), 3.87 (1H, m), 4.36 (1H, m), 4.50-4.65 (1H, m), 6.31 (1H, dd), 6.42 (1H, d), 7.36 (2H, d), 7.43 (2H, d), 8.43 (1H, m), 9.60-10.20 (1H, m) MS: 319 |
| 75 | 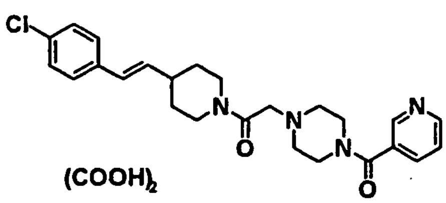 $(COOH)_2$ | MS: 453 |
| 76 | 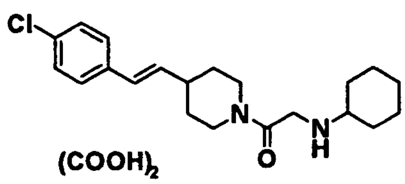 $(COOH)_2$ | NMR: 1.00-1.45 (7H, m), 1.60 (1H, m), 1.70-1.85 (4H, m), 2.04 (2H, m), 2.44 (1H, m), 2.77 (1H, m), 2.89 (1H, m), 3.10 (1H, m), 3.77 (1H, m), 3.95-4.05 (2H, m), 4.37 (1H, m), 6.29 (1H, dd), 6.41 (1H, d), 7.36 (2H, d), 7.43 (2H, d) MS: 361 |

Table 17

| Ex. | STRUCTURE | DATA |
|---|---|---|
| 77 | 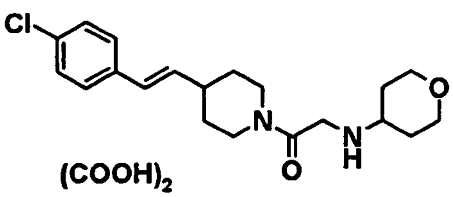 $(COOH)_2$ | MS: 363 |

(continued)

| Ex. | STRUCTURE | DATA |
|---|---|---|
| 78 | 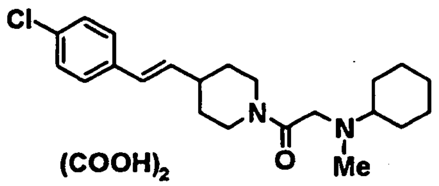 | NMR: 1.00-1.50 (7H, m), 1.60 (1H, m), 1.80 (4H, m), 1.98 (2H, m), 2.44 (1H, m), 2.66 (3H, s), 2.77 (1H, m), 3.00-3.15 (2H, m), 3.79 (1H, m), 4.10 (2H, m), 4.37 (1H, m), 6.30 (1H, dd), 6.41 (1H, d), 7.36 (2H, d), 7.43 (2H, d) MS: 375 |
| 79 | 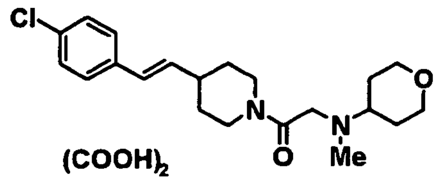 | MS: 377 |
| 80 | 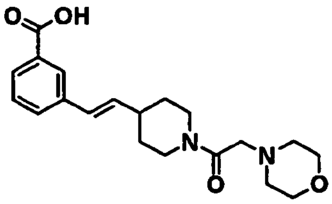 | MS: 359 |
| 81 | 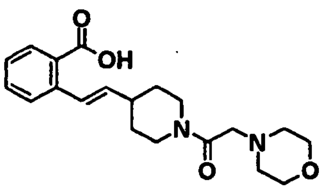 | MS: 359 |
| 82 | 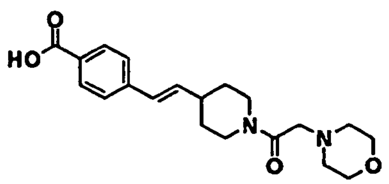 | MS: 359 |

Table 18

| Ex. | STRUCTURE | DATA |
|---|---|---|
| 83 | 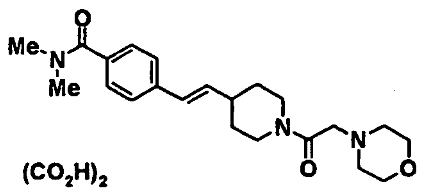 | MS: 386 |
| 84 | 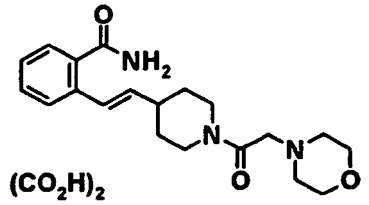 | MS: 358 |

(continued)

| Ex. | STRUCTURE | DATA |
|---|---|---|
| 85 | 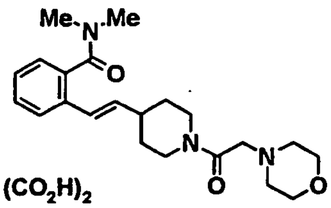 $(CO_2H)_2$ | MS: 386 |
| 86 | 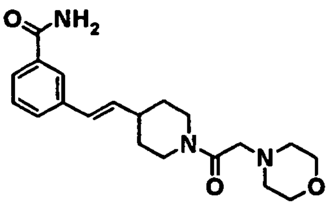 | MS: 358 |
| 87 | 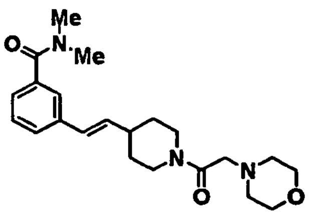 | MS:386 |
| 88 | 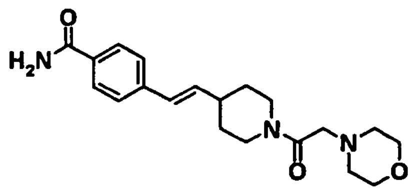 | MS: 358 |

Table 19

| Ex. | STRUCTURE | DATA |
|---|---|---|
| 89 | 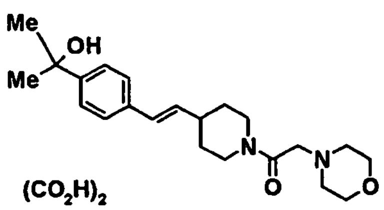 $(CO_2H)_2$ | MS: 373 |
| 90 | 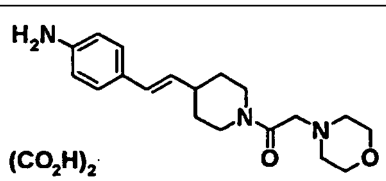 $(CO_2H)_2$ | MS: 330 |
| 91 | 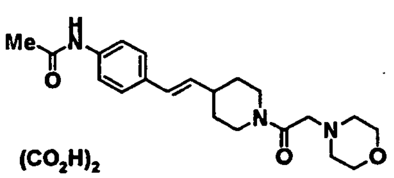 $(CO_2H)_2$ | MS: 372 |

Table 20

| | | |
|---|---|---|
| 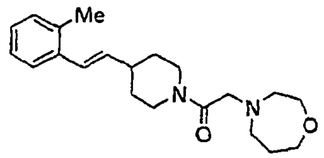 | 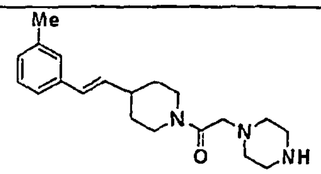 | 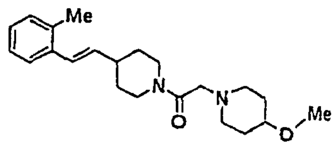 |
| 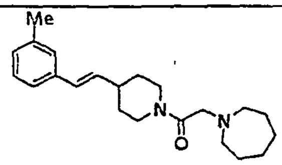 | 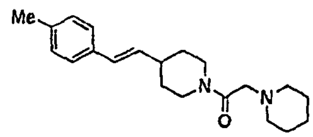 | 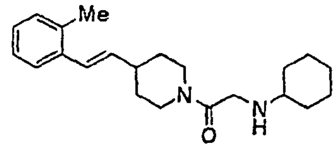 |
| 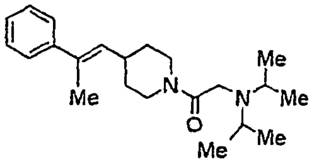 | 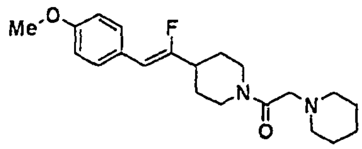 | 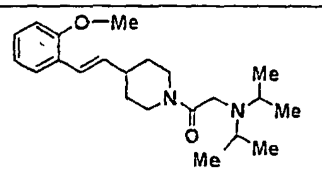 |
| 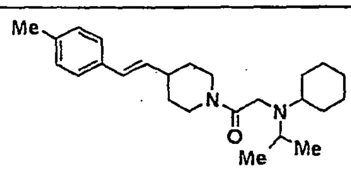 | | 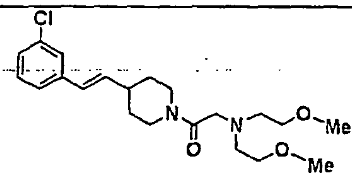 |
| 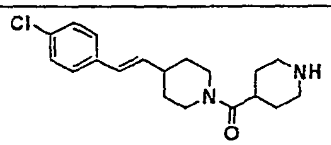 | 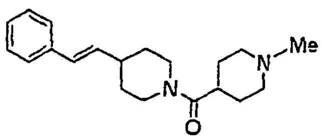 | 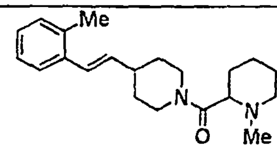 |
| 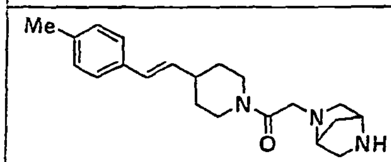 | | 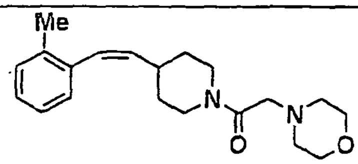 |
| | | |
| 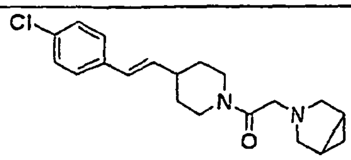 | 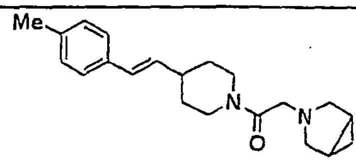 | 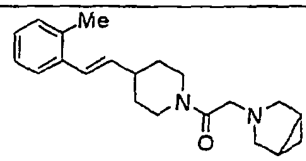 |

[Pharmacological tests]

For the compound (I) of the present invention, sodium channel inhibition and analgesic actions in animal models

were tested. The tests are described in detail below.

(Sodium channel inhibition test)

The sodium channel inhibition actions of representative compounds of the present compound (I) were confirmed by a [$^{14}$C] guanidine uptake test using a rat brain tissue. The [$^{14}$C] guanidine uptake test was conducted by modifying the method of Bonisch et al. (British Journal of Pharmacology 108, 436-442, 1993). The [$^{14}$C] guanidine was used as a sodium tracer, and the inhibitory activity on uptake of [$^{14}$C] guanidine induced by veratridine as a sodium channel activator into rat cerebral cortex primary neurons was measured.

a. Preparation of rat cerebral cortex primary neuron culture system.

A pregnant rat (Wistar, female, pregnancy = 19 days old) was anesthetized with diethyl ether and killed by bleeding with the cutting of the carotid artery. Fetuses were excised from the pregnant rat and sterilized with ethanol for disinfection. Then, from the fetuses was dissociated the cerebral cortex. The cerebral cortex was digested with papain and dispersed in a culture medium. Then, the dissociated neurons were placed in a 96-well white plate coated with a poly-L-lysine, at a density of $2.5 \times 10^6$ cells/well, and cultured for 2 days in a $CO_2$ incubator (37˚C, 5% $CO_2$).

b. Evaluation of test compounds

Each well was washed once with an assay buffer (135 mM choline Cl, 5 mM KCl, 1 mM $MgSO_4$, 5.5 mM glucose, 1 mg/mL BSA, 10 mM Hepes-Tris, pH 7.4). The assay buffer was added to each well and incubated at 25˚C for 10 minutes. Then, the assay buffer was replaced by a reaction solution (test compound, [$^{14}$C] guanidine and 100 μM veratridine) and incubated at 25˚C for 15 minutes. The reaction was terminated by three times washing with a cold-washing buffer (135 mM NaCl, 5 mM KCl, 1 mM $MgSO_4$, 10 mM Hepes-Tris, pH 7.4). To each well was added 17 μL of 0.1 N NaOH; stirring was made; then, 100 μL of a scintillator was added; and the radioactivity of each well was measured using a liquid scintillator counter. In each test, the uptake amount of [$^{14}$C] guanidine inhibited by 1 mM of mexiletine was taken as the portion of the specific uptake via sodium channel. The activity of test compound on sodium channel is expressed by 50% inhibitory concentration ($IC_{50}$) for the specific uptake.

As shown in Table 21, the present compound include compounds showing $IC_{50}$ values of about 3 to 30 μM and have higher effects than mexiletine (about 70 μM).

Table 21

| Ex. | I $C_{50}$ (μM) | Ex. | I $C_{50}$ (μM) |
|---|---|---|---|
| 1 | 27 | 10 | 16 |
| 26 | 25 | 37 | 13 |
| 40 | 23 | 50 | 12 |
| 52 | 24 | 65 | 12 |
| 68 | 25 | 74 | 13 |
| 76 | 3.4 | 78 | 3.1 |

(Analgesic action on diabetic neuropathy in streptozotocin-induced diabetic mice)

Representative compounds of Compound (I) of the present invention were assessed for the analgesic action on diabetic neuropathy in streptozotocin (STZ)-induced diabetic mice to confirm the effect on neuropathic pain. The assessment was performed by the method of Kamei et al. (Pharmacology Biochemistry & Behavior 39, 541-544, 1991) with some modifications.

Male ICR mice of 4 weeks old were intraperitoneally injected with 200 mg/kg weight of STZ to prepare mice with diabetic neuropathy. The analgesic action was assessed by the tail pinch test. Specifically, the analgesic action was detected as prolongation in withdrawal latency (in seconds), i.e., the time until the animal showed head-turning response after the tail was pinched with forceps. On Day 14 following the STZ injection, a pretest was carried out to determine the response latency before the administration of a test compound. Only the animals showing not longer than 3 seconds of the response latency in the pretest were used for the test compound assessment on the following day

(Day 15 after STZ injection). In the test compound assessment, the response latency after the administration of a test compound was measured. The test compound was orally administered at a dose of 30 mg/kg, 45 minutes prior to the response latency measurement. The analgesic action of a test compound is expressed as prolongation of latency (in seconds) calculated by the formula:

(response latency after administration of a test compound) - (response latency before administration of a test compound).

As shown in Table 22, the present compounds tested showed a prolongation of latency, of about 2 to 4 seconds and had good analgesic actions.

Table 22

| Ex. | Increase in latent time (sec) | Ex. | Increase in latent time (sec) |
|---|---|---|---|
| 3 | 2.3 | 26 | 2.3 |
| 40 | 3.4 | 76 | 3.2 |
| 78 | 2.4 | | |

It was confirmed by the above test that the present compound has a sodium channel inhibition activity higher than that of mexiletine. It was also confirmed that the present compound, when orally administered, shows a good analgesic action in an animal model of morbid state, that is, in mice of diabetic neuropathy. Thus, the present compound was confirmed to be effective as a superior sodium channel inhibitor to pain, particularly to neuropathic pain accompanied with diabetic neuropathy, etc.

(Examination on separation from side effect)

With drugs currently used for cure of neuropathic pain, the difference between the dose for expression of analgesic action and the dose at side effect expression is small and, therefor, the side effect appears frequently, making difficult the use at a high dose. It was confirmed by the rotarod test often used as a classical method for detection of side effect that representative compounds of the compound of the present invention hardly cause side effect even when administered at a dose considerably higher than the dose required for expression of analgesic action. The examination of separation from side effect dose was conducted by partially modifying the method of Christensen et al. (Pain 93, 147-153, 2001). Male SD rats were used in the examination. In each test run, each test animal was placed on the apparatus which was accelerated from 4 rpm to 40 rpm at a constant acceleration in 5 minutes, and there was measured a time (holding time, sec) up to the dropping of the test animal. On the day of test run, first, each test animal was measured for body weight and subjected to three test runs before drug administration. There were selected, for examination of pharmacological action, those test animals which showed the longest holding time of 90 seconds or longer in the three test runs. These selected test animals were divided into groups so that the difference in average of holding times before drug administration among groups became minimum. Each drug was administered orally together with a solvent (5 ml/kg). After the drug administration, two test runs were conducted at the same timing as for the test for measurement of an anti-allodynia effect in L5/L6 spinal nerves-ligated rat. For example, when the anti-allodynia effect was measured 30 minutes after drug administration, the test runs after drug administration were conducted 30 minutes after drug administration, also in the rotarod test. As the holding time after drug administration, of each test animal, an average of two test runs was adopted. The holding time of each group was expressed as average $\pm$ standard error. Significant difference between solvent-administered group and drug-administered group was analysed using Dunnet's test and the level of $p < 0.05$ was judged as significant.
The compounds of the present invention included compounds having such a property that the side effect was hardly caused even when administered at a dose considerably higher than the dose effective in the test for measurement of the anti-allodynia effect in rats with L5/L6 spinal nerve ligation.

(Anti-allodynia effect in rats with L5/L6 spinal nerve ligation)

One of the major symptoms in neuropathic pain is a markedly lowered threshold of response to tactile stimulation (allodynia). The anti-allodynia effect of the representative compounds in the compounds of the present invention was confirmed by assessing the analgesic action in L5/L6 spinal nerve ligation rats. The assessment was performed by the method of Kim and Chung (Pain 50, 355-363, 1992) with some modifications. Under pentobarbital anesthesia, male SD rats of 5 or 6 weeks old were given surgery to ligate both the left L5 and L6 lumbar spinal nerves tightly with silk threads. For the assessment of analgesic action, the von Frey hair test was adopted. That is, the animal's hindpaw was picked with hair and the lowest strength of hair for limb withdrawal response was designated as a response threshold (log gram) to mechanical stimulation. Since it was confirmed in prior test that the response threshold of the animal's hindpaw ipsilateral to the side of ligation was markedly low during days 7 to 14 after the surgery (in the state of allodynia), the anti-allodynia effect of a test compound was assessed on any day between days 7 and 14 from the surgery. On the day before the assessment of a test compound, the response threshold before administration of a test compound was measured. The animals were divided into 4 to 5 groups so that differences in the mean values of response thresholds before administration among the groups and variation within groups become small. In the assessment of a test compound, the response threshold after administration of the test compound was measured. The test compound was orally given 30 minutes before measurement of the response threshold. The anti-allodynia action potency of a test compound is expressed as $ED_{50}$. In the calculation of the $ED_{50}$, the thresholds of ipsilateral and contralateral paws in the solvent group were designated as 0% and 100%, respectively.
The present compound included those compounds showing an excellent $ED_{50}$. Meanwhile, the $ED_{50}$ of mexiletine was about 70 mg/kg.

## Claims

**1.** A piperidine derivative represented by Formula (I) or a pharmaceutically acceptable salt thereof:

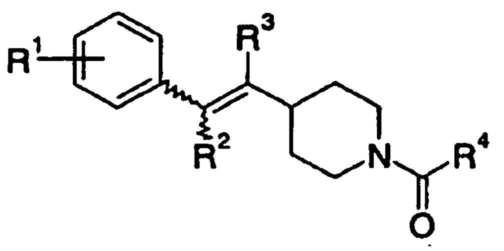

(I)

wherein $R^1$ is a hydrogen atom, halogen atom, $C_{1-6}$ alkyl which may be substituted, -O-$C_{1-6}$ alkyl which may be substituted, -O-aryl, aryl, cycloalkyl, -C(=O)-$C_{1-6}$ alkyl, -COOH, -C(=O)-O-$C_{1-6}$ alkyl, -C(=O)-$NH_2$, -C(=O)NH-$C_{1-6}$ alkyl, -C(=O)N-($C_{1-6}$ alkyl) $_2$, -OH, -O-C(=O)-$C_{1-6}$ alkyl, $NH_2$, -NH-$C_{1-6}$ alkyl, -N-($C_{1-6}$ alkyl)$_2$, -NH-C(=O)-$C_{1-6}$ alkyl, -CN or -$NO_2$; $R^2$ and $R^3$ may be the same or different and are each a hydrogen atom, $C_{1-6}$ alkyl or halogen atom; and $R^4$ is one of mono-valent groups (a), (b) and (c):

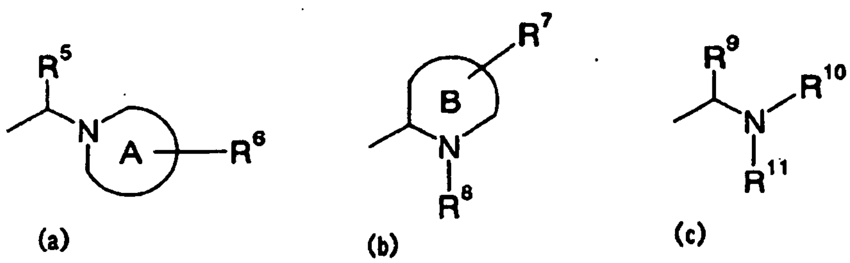

(a) (b) (c)

wherein A and B are each a nitrogen-containing heterocyclic ring; $R^5$ and $R^8$ to $R^{11}$ may be the same or different and are each a hydrogen atom, $C_{1-6}$ alkyl, -C(=O)-O-$C_{1-6}$ alkyl which may be substituted, $C_{1-6}$ alkylene-O-$C_{1-6}$ alkyl, cycloalkyl or a saturated or unsaturated 5- or 6-membered heterocyclic ring having 1 to 3 hetero-atoms selected

from N, S and O;$R^6$ is hydrogen atom, $C_{1-6}$ alkyl, -O-$C_{1-6}$ alkyl, -O-$C_{1-6}$ alkylene-O- which is bonded with one carbon atom of A ring to form a ring, -C(=O)-O-$C_{1-6}$ alkyl which may be substituted, -OH, -$C_{1-6}$ alkylene-OH, or -C(=O)-heteroaryl; and $R^7$ is hydrogen atom, $C_{1-6}$ alkyl, -O-$C_{1-6}$ alkyl, -C(=O)-O-$C_{1-6}$ alkyl, -OH, -$C_{1-6}$ alkylene-OH or -C(=O)-heteroaryl.

**2.** A piperidine derivative or a pharmaceutically acceptable salt thereof according to Claim 1, wherein $R^4$ is the monovalent group (a) and the nitrogen-containing heterocyclic ring represented by A is a pyrrolidine, piperidine, morpholine, piperazine or oxazepam ring.

**3.** A piperidine derivative or a pharmaceutically acceptable salt thereof according to Claim 1, wherein $R^4$ is the monovalent group (b) and the nitrogen-containing heterocyclic ring represented by B is a pyrrolidine or piperidine ring.

**4.** A piperidine derivative or a pharmaceutically acceptable salt thereof according to Claim 1, wherein $R^4$ is the monovalent group (c) and $R^9$ to $R^{11}$ may be the same or different and are each hydrogen atom, $C_{1-6}$ alkyl, -C(=O)-O-$C_{1-6}$ alkyl which may be substituted, $C_{1-6}$ alkylene-O-$C_{1-6}$ alkyl, cycloalkyl or a saturated or unsaturated 5- or 6-membered heterocyclic ring group having 1 to 3 hetero-atoms selected from N, S and O.

**5.** A piperidine derivative or a pharmaceutically acceptable salt thereof according to Claim 4, wherein at least one of $R^9$ to $R^{11}$ in the group (c) is cycloalkyl and the other(s) may each independently be a hydrogen atom, $C_{1-6}$ alkyl, -C (=O) -O-$C_{1-6}$ alkyl which may be substituted, lower $C_{1-6}$-O-$C_{1-6}$ alkyl, cycloalkyl or a saturated or unsaturated 5- or 6-membered heterocyclic ring group having 1 to 3 hetero-atoms selected from N, S and O.

**6.** A piperidine derivative or a pharmaceutically acceptable salt thereof according to Claim 1, wherein the piperidine derivative represented by the Formula (I) is:

4-(2-{4-[(E)-2-(2-methylphenyl)vinyl]piperidin-1-yl}-2-oxoethyl)morpholine;
4-(2-{4-[(E)-2-(4-isopropylphenyl)vinyl]piperidin-1-yl}-2-oxoethyl)morpholine;
4-(2-{4-[(E)-2-(4-chlorophenyl)vinyl]piperidin-1-yl}-2-oxoethyl)morpholine;
N-(2-{4-[(E)-2-(4-chlorophenyl)vinyl]piperidin-1-yl}-2-oxoethyl)cyclohexaneamine; or
N-(2-{4-[(E)-2-(4-chlorophenyl)vinyl]piperidin-1-yl}-2-oxoethyl)-N-methylcyclohexaneamine.

**7.** A composition containing a piperidine derivative or a pharmaceutically acceptable salt thereof according to any preceding claim and a pharmaceutically acceptable carrier.

**8.** A composition according to Claim 7 for use in the treatment of pain.

### Patentansprüche

**1.** Piperidinderivat der Formel (I) oder ein pharmazeutisch akzeptables Salz davon:

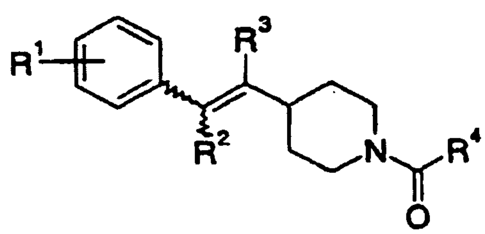

(I)

worin $R^1$ ein Wasserstoffatom, Halogenatom, $C_{1-6}$-Alkyl, das substituiert sein kann, -O-$C_{1-6}$-Alkyl, das substituiert sein kann, -O-Aryl, Aryl, Cycloalkyl, -C(=O)-$C_{1-6}$-Alkyl, -COOH, -C(=O)-O-$C_{1-6}$-Alkyl, -C(=O)-$NH_2$, -C(=O)NH-$C_{1-6}$-Alkyl, -C(=O)N-($C_{1-6}$-Alkyl)$_2$, -OH, -O-C(=O)-$C_{1-6}$-Alkyl, -$NH_2$, -NH-$C_{1-6}$-Alkyl, -N-($C_{1-6}$-Alkyl)$_2$, -NH-C(=O)-$C_{1-6}$-Alkyl, -CN oder -$NO_2$ ist; $R^2$ und $R^3$ können gleich oder verschieden voneinander sein und sind jeweils ein Wasserstoffatom, $C_{1-6}$-Alkyl oder ein Halogenatom; und $R^4$ ist eine der monovalenten Gruppen (a), (b) oder (c):

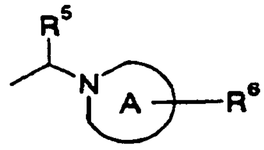
(a)

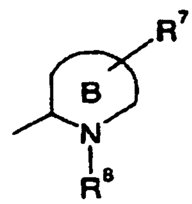
(b)

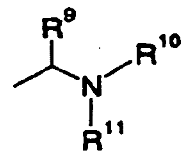
(c)

worin A und B jeweils ein stickstoffhaltiger heterocyclischer Ring ist; $R^5$ und $R^8$ bis $R^{11}$ können gleich oder verschieden voneinander sein und sind jeweils ein Wasserstoffatom, $C_{1-6}$-Alkyl, -C(=O)-O-$C_1$-$_6$-Alkyl, das substituiert sein kann, $C_1$-$_6$-Alkylen-O-$C_1$-$_6$-alkyl, Cycloalkyl oder ein gesättigter oder ungesättigter 5- oder 6-gliedriger heterocyclischer Ring mit 1 bis 3 Heteroatomen, die ausgewählt sind aus N, S oder O; $R^6$ ein Wasserstoffatom, $C_1$-$_6$-Alkyl, -O-$C_1$-$_6$-Alkyl, -O-$C_1$-$_6$-Alkylen-O-, das an ein Kohlenstoffatom des A-Rings zur Bildung eines Rings gebunden ist, -C(=O)-O-$C_1$-$_6$-Alkyl, das substituiert sein kann, -OH, -$C_1$-$_6$-Alkylen-OH oder -C(=O)-Heteroaryl ist; und $R^7$ ein Wasserstoffatom, $C_1$-$_6$-Alkyl, -O-$C_1$-$_6$-Alkyl, -C(=O)-O-$C_1$-$_6$-Alkyl, -OH, -$C_1$-$_6$-Alkylen-OH oder -C(=O)-Heteroaryl ist.

2. Piperidinderivat oder ein pharmazeutisch akzeptables Salz davon gemäß Anspruch 1, worin $R^4$ die monovalente Gruppe (a) ist und der stickstoffhaltige heterocyclische Ring, der durch A dargestellt ist, ein Pyrrolidin-, Piperidin-, Morpholin-, Piperazin- oder ein Oxazepamring ist.

3. Piperidinderivat oder ein pharmazeutisch akzeptables Salz davon gemäß Anspruch 1, worin $R^4$ die monovalente Gruppe (b) ist und der stickstoffhaltige heterocyclische Ring, der durch B dargestellt ist, ein Pyrrolidin- oder ein Piperidinring ist.

4. Piperidinderivat oder ein pharmazeutisch akzeptables Salz davon gemäß Anspruch 1, worin $R^4$ die monovalente Gruppe (c) ist und $R^9$ bis $R^{11}$ gleich oder verschieden voneinander sind und jeweils ein Wasserstoffatom, $C_1$-$_6$-Alkyl, -C(=O)-O-$C_1$-$_6$-Alkyl, das substituiert sein kann, $C_1$-$_6$-Alkylen-O-$C_1$-$_6$-alkyl, Cycloalkyl oder eine gesättigt oder ungesättigte 5- oder 6-gliedrige heterocyclische Ringgruppe mit 1 bis 3 Heteroatomen ist, die ausgewählt sind aus N, S oder O.

5. Piperidinderivat oder ein pharmazeutisch akzeptables Salz davon gemäß Anspruch 4, worin mindestens einer von $R^9$ bis $R^{11}$ in der Gruppe (c) Cycloalkyl ist und die anderen jeweils unabhängig voneinander ein Wasserstoffatom, $C_1$-$_6$-Alkyl, -C(=O)-O-$C_1$-$_6$-Alkyl, das substituiert sein kann, niedriges $C_1$-$_6$-O-$C_1$-$_6$-Alkyl, Cycloalkyl oder eine gesättigte oder ungesättigte 5- oder 6-gliedrige heterocyclische Ringgruppe mit 1 bis 3 Heteroatomen ist, die ausgewählt sind aus N, S und O.

6. Piperidinderivat oder ein pharmazeutisch akzeptables Salz davon gemäß Anspruch 1, worin das Piperidinderivat der Formel (I) folgendes ist:

4-(2-{4-[(E)-2-(2-Methylphenyl)vinyl]piperidin-1-yl}-2-oxoethyl)morpholin;
4-(2-{4-[(E)-2-(4-Isopropylphenyl)vinyl]piperidin-1-yl}-2-oxoethyl)morpholin;
4-(2-{4-[(E)-2-(4-Chlorophenyl)vinyl]piperidin-1-yl}-2-oxoethyl)morpholin;
N-(2-{4-[(E)-2-(4-Chlorophenyl)vinyl]piperidin-1-yl}-2-oxoethyl)cyclohexanamin; oder
N-(2-{4-[(E)-2-(4-Chlorophenyl)vinyl]piperidin-1-yl}-2-oxoethyl)-N-methylcyclohexanamin.

7. Zusammensetzung, die ein Piperidinderivat oder ein pharmazeutisch akzeptables Salz davon gemäß irgendeinem der vorangehenden Ansprüche und einen pharmazeutisch akzeptablen Träger enthält.

8. Zusammensetzung gemäß Anspruch 7 zur Verwendung in der Behandlung von Schmerz.

## Revendications

1. Dérivé de pipéridine représenté par la formule (I) ou sel pharmaceutiquement acceptable de celui-ci :

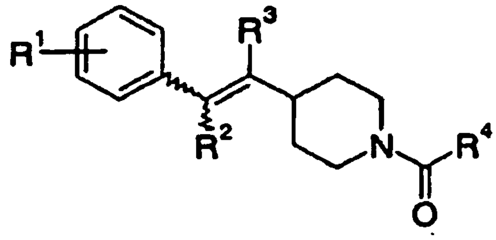

(I)

où $R^1$ est un atome d'hydrogène, un atome d'halogène, un $C_{1-6}$ alkyle qui peut être substitué, un -O-$C_{1-6}$ alkyle qui peut être substitué, un -O-aryle, un aryle, un cycloalkyle, un -C(=O)-$C_{1-6}$ alkyle, -COOH, un -C(=O)-O-$C_{1-6}$ alkyle, -C(=O)-$NH_2$, un -C(=O) NH-$C_{1-6}$ alkyle, un -C(=O)N-($C_{1-6}$ alkyl)$_2$, un -OH, -O-C(=O)-$C_{1-6}$ alkyle, -$NH_2$, un -NH-$C_{1-6}$ alkyle, un -N-($C_{1-6}$ alkyl)$_2$, un -NH-C(=O)-$C_{1-6}$ alkyle, -CN ou -$NO_2$ ; $R^2$ et $R^3$ peuvent être identiques ou différents et sont chacun un atome d'hydrogène, un $C_{1-6}$ alkyle ou un atome d'halogène ; et $R^4$ est l'un parmi les groupes monovalents (a), (b) et (c) :

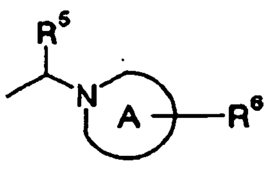

(a)

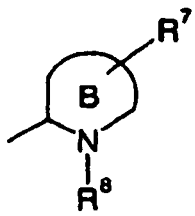

(b)

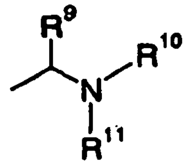

(c)

où A et B sont chacun un anneau hétérocyclique contenant un azote ; $R^5$ et $R^8$ à $R^{11}$ peuvent être identiques ou différents et sont chacun un atome d'hydrogène, un $C_{1-6}$ alkyle, un -C(=O)-O-$C_{1-6}$ alkyle qui peut être substitué, un $C_{1-6}$ alkylène-O-$C_{1-6}$ alkyle, un cycloalkyle ou un anneau hétérocyclique à 5 ou 6 chaînons saturé ou insaturé ayant 1 à 3 hétéroatomes choisis parmi N, S et O ; $R^6$ est un atome hydrogène, un $C_{1-6}$ alkyle, un -O-$C_{1-6}$ alkyle, un -O-$C_{1-6}$ alkylène-O- qui est lié avec un atome de carbone du cycle A pour former un cycle, un -C (=O)-O-$C_{1-6}$ alkyle qui peut être substitué, -OH, un -$C_{1-6}$ alkylène-OH ou un -C(=O)-hétéroaryle ; et $R^7$ est un atome d'hydrogène, un $C_{1-6}$ alkyle, un -O-$C_{1-6}$ alkyle, un -C(=O)-O-$C_{1-6}$ alkyle, -OH, un -$C_{1-6}$ alkylène-OH ou un -C(=O)-hétéroaryle.

**2.** Dérivé de pipéridine ou sel pharmaceutiquement acceptable de celui-ci selon la revendication 1, où $R^4$ est le groupe monovalent (a) et l'anneau hétérocyclique contenant un azote représenté par A est un cycle pyrrolidine, pipéridine, morpholine, pipérazine ou oxazépam.

**3.** Dérivé de pipéridine ou sel pharmaceutiquement acceptable de celui-ci selon la revendication 1, où $R^4$ est le groupe monovalent (b) et l'anneau hétérocyclique contenant un azote représenté par B est un cycle pyrrolidine ou pipéridine.

**4.** Dérivé de pipéridine ou sel pharmaceutiquement acceptable de celui-ci selon la revendication 1, où $R^4$ est le groupe monovalent (c) et $R^9$ à $R^{11}$ peuvent être identiques ou différents et sont chacun un atome d'hydrogène, un $C_{1-6}$ alkyle, un -C(=O)-O-$C_{1-6}$ alkyle qui peut être substitué, un $C_{1-6}$ alkylène-O-$C_{1-6}$ alkyle, un cycloalkyle ou un groupe à anneau hétérocyclique à 5 ou 6 chaînons saturé ou insaturé ayant 1 à 3 hétéroatomes choisis parmi N, S et O.

**5.** Dérivé de pipéridine ou sel pharmaceutiquement acceptable de celui-ci selon la revendication 4, dans lequel au moins l'un parmi $R^9$ à $R^{11}$ dans le groupe (c) est un cycloalkyle et le ou les autres peuvent être chacun indépendamment un atome d'hydrogène, un $C_{1-6}$ alkyle, un -C(=O)-O-$C_{1-6}$ alkyle qui peut être substitué, un $C_{1-6}$-O-$C_{1-6}$ alkyle inférieur, un cycloalkyle ou un groupe à anneau hétérocyclique à 5 ou 6 chaînons saturé ou insaturé ayant 1 à 3 hétéroatomes choisis parmi N, S et O.

**6.** Dérivé de pipéridine ou sel pharmaceutiquement acceptable de celui-ci selon la revendication 1, lequel dérivé de

pipéridine représenté par la formule (I) est :

4-(2-{4-[(*E*)-2-(2-méthylphényl)vinyl]pipéridine-1-yl}-2-oxoéthyl)morpholine ;
4-(2-{4-[(*E*)-2-(4-isopropylphényl)vinyl]pipéndine-1-yl}-2-oxoéthyl)morpholine ;
4-(2-{4-[(*E*)-2-(4-chlorophényl)vinyl]pipéridine-1-yl}-2-oxoéthyl)morpholine ;
N-(2-{4-[(*E*)-2-(4-chlorophényl)vinyl]pipéridine-1-yl}-2-oxoéthyl)cyclohexaneamine ; ou
N-(2-{4-[(*E*-2-(4-chlorophényl)vinyl]pipéridine-1-yl}-2-oxoéthyl)-*N*-méthylcyclohexaneamine.

**7.** Composition contenant un dérivé de pipéridine ou un sel pharmaceutiquement acceptable de celui-ci selon l'une quelconque des revendications précédentes et support pharmaceutiquement acceptable.

**8.** Composition selon la revendication 7 destinée à une utilisation dans le traitement de la douleur.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0153288 A
- WO 9413291 A
- WO 9719059 A


### Non-patent literature cited in the description


- **Goldin AL.** Annals of the New York Academy of Sciences. 1999, vol. 868, 38-50
- **Goldin AL.** *Annual Review of Physiology,* 2001, vol. 63, 871-894
- **Taylor CP.** *Current Pharmaceutical Design,* 1996, vol. 2, 375-388
- *Prog. Med.,* 1985, vol. 5, 2157-2161
- Molecular Design. Development of Drugs. Hirokawa Shoten, 1990, vol. 7, 163-198
- *Org. React.,* 1965, vol. 14, 270-490
- Protective Group in Organic Synthesis. JOHN WILEY & SONS, INC,
- *Org. Synth. III,* 1955, vol. 200
- *Org. React.,* 1964, vol. 6, 339-366
- *Org. React.,* 1967, vol. 8, 258-304
- *Org. React.,* 1990, vol. 38, 1-223
- *J. Am. Chem. Soc.,* 1955, vol. 77, 3378
- *J. Am. Chem. Soc.,* 1977, vol. 99, 2805
- *Synthesis,* 1973, 457
- *Tetrahedron,* 1974, vol. 30, 3817
- *Org. React.,* 1968, vol. 16, 1-438
- *Org. Synth.,* 1955, vol. III, 200
- **Bonisch et al.** *British Journal of Pharmacology,* 1993, vol. 108, 436-442
- **Kamei et al.** *Pharmacology Biochemistry & Behavior,* 1991, vol. 39, 541-544
- **Christensen et al.** *Pain,* 2001, vol. 93, 147-153
- **Kim ; Chung.** *Pain,* 1992, vol. 50, 355-363